(19) **Europäisches Patentamt** / **European Patent Office** / **Office européen des brevets**

(11) **EP 4 471 151 A1**

(12) **EUROPEAN PATENT APPLICATION**
published in accordance with Art. 153(4) EPC

(43) Date of publication:
**04.12.2024 Bulletin 2024/49**

(21) Application number: **23746091.0**

(22) Date of filing: **16.01.2023**

(51) International Patent Classification (IPC):
*C12N 15/867* (2006.01)      *C12N 15/47* (2006.01)
*C12N 15/62* (2006.01)      *C12N 7/01* (2006.01)
*A61K 39/205* (2006.01)      *A61K 47/68* (2017.01)
*A61K 48/00* (2006.01)      *A61P 31/14* (2006.01)

(52) Cooperative Patent Classification (CPC):
A61K 39/00; A61K 39/205; A61K 47/68;
A61K 48/00; A61P 31/14; A61P 35/00;
C07K 14/145; C07K 19/00; C12N 5/10; C12N 7/00;
C12N 15/62; C12N 15/867; Y02A 50/30

(86) International application number:
**PCT/CN2023/072446**

(87) International publication number:
**WO 2023/143209 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  25.01.2022   CN 202210089576
24.03.2022   CN 202210298203

(71) Applicant: **Sunshine Lake Pharma Co., Ltd.**
**Dongguan, Guangdong 523000 (CN)**

(72) Inventors:
• **YANG, Xiaodan**
 **Dongguan, Guangdong 523871 (CN)**
• **CHEN, Shiyou**
 **Dongguan, Guangdong 523871 (CN)**
• **ZHU, Xiuqin**
 **Dongguan, Guangdong 523871 (CN)**

• **LI, Linsheng**
 **Dongguan, Guangdong 523871 (CN)**
• **DONG, Junji**
 **Dongguan, Guangdong 523871 (CN)**
• **ZHAO, Guanghui**
 **Dongguan, Guangdong 523871 (CN)**
• **LI, Lijia**
 **Dongguan, Guangdong 523871 (CN)**
• **CHEN, Xiaofeng**
 **Dongguan, Guangdong 523871 (CN)**
• **LI, Wenjia**
 **Dongguan, Guangdong 523871 (CN)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **VIRAL VECTOR AND APPLICATION THEREOF**

(57)      Provided is a set of viral vectors, comprising: a first viral vector that carries a first nucleic acid molecule encoding an envelope protein, and a second viral vector that carries a second nucleic acid molecule encoding a fusion protein, the fusion protein comprising: a single-chain antibody capable of binding to CD28 or CD3, and a C-terminal domain, comprising a transmembrane region and an intracellular region, of the envelope protein. The C terminal of the single-chain antibody is connected to the N terminal of the C-terminal domain of the envelope protein, and the envelope protein and the fusion protein are in a non-fusion form. Also provided are a method for obtaining a lentivirus and an obtained lentivirus, a method for introducing a lentivirus into an unactivated T lymphocyte, a method for expressing a target gene, a method for obtaining a CAR-T cell and an obtained CAR-T cell, a pharmaceutical composition, and a use thereof.

EP 4 471 151 A1

## Description

### FIELD

The present invention relates to the field of biotechnology, specifically the present invention relates to viral vectors and uses thereof, and more specifically the present invention relates to viral vectors, a method for obtaining lentivirus, lentivirus, a method for introducing lentivirus into unactivated T lymphocytes, a method for expressing gene of interest, and a method for obtaining CAR-T cells.

### BACKGROUND

Gene therapy aims to treat or cure diseases by modifying the gene expression of organisms, it is divided into *In vivo* gene therapy and *Ex vivo* gene therapy according to the location where gene transmission occurs. The in vivo therapy strategy is to assemble the therapeutic gene into a specific vector and transduce the therapeutic gene into cells through the vector in a human body. Currently, adeno-associated viral (AAV) vectors are mainly used. The ex vivo therapy strategy is to separate the cells of a patient, genetically modify the cells during ex vivo culture, and then inject the genetically modified cells back into the patient. Currently, lentiviral vectors (LV) are mainly used.

Comparing the in vivo and ex vivo therapy strategies, we can know that the drug of the former is transgenic vector, which is a general-purpose product, and the production process of the product only involves the production of transgenic vector. The drug of the latter is modified patient cells, which is a personalized product and is only suitable for the treatment of diseases that the blood cells can be separated and cultured ex vivo. The production process of the product is complex, involving the production of transgenic vector, the isolation of patient cells, ex vivo culture, cell genetic modification, and cell reinfusion. In general, the in vivo gene therapy products have the huge advantages of wide indications, relatively simple and universal production process, mass production, and low cost.

However, the current in vivo gene therapy using AAV vectors has certain problems in terms of effectiveness and safety. Regarding effectiveness, after the gene is transduced into the cells of a patient, it is free from the host genome in the form of a microloop and will be lost as the cells divide and undergo apoptosis, resulting in the gradual weakening of the drug efficacy. Regarding safety, AAV gene therapy has experienced frequent deaths during its introduction to the market and in early clinical trials, and FDA has focused on the safety issues of hepatotoxicity, kidney damage, and neuronal loss of this therapy. In addition, the industry once believed that AAV transduced genes do not integrate into the host genome and do not pose a risk of carcinogenesis and are highly safe. However, there are continuous reports that AAV transduced genes will integrate into the host genome and have carcinogenicity risks.

Genes transduced by lentiviral vectors can penetrate the nuclear membrane and can be efficiently integrated into the host genome in both dividing and non-dividing cells, allowing the therapeutic genes to replicate as cells divide and persist and stably exist in the transduced cells, which can achieve a one-time administration and a lifelong cure. At present, lentiviral vectors are widely used in ex vivo gene therapy such as CAR-T cells production and hematopoietic stem cell modification. A large amount of preclinical and clinical data has been accumulated, and there are no reports of tumors caused by lentiviral vector transduced genes inserted into the host genome, proving the safety of lentivirus. However, lentiviral vectors have not yet made breakthroughs in in vivo gene therapy, the main reason is that the transduction is non-targeted and may affect safety due to off-target. Constructing targeted lentiviral vectors can improve the corresponding safety. Some articles have reported that linking scFv or pre-designed ankyrin repeat protein (DARPin) to the envelope protein of lentiviral vectors can enhance the transduction efficiency of the vector for certain types of cells, but there are still problems that the modified vectors have a high off-target rate or are not evaluated, and the production titer is greatly reduced, resulting in the inability to be used in industrial production.

Therefore, new technologies are needed to be developed to solve the above problems.

### SUMMARY OF THE INVENTION

The present invention aims to solve one of the technical problems in the related art at least to a certain extent. To this end, the present invention provides a viral vector with targeted infectivity and significantly improved virus titer.

In the first aspect of the present invention, provided herein is a group of viral vectors. According to the embodiments of the present invention, the group of viral vectors comprises: a first viral vector, the first viral vector carries a first nucleic acid molecule, and the first nucleic acid molecule encodes an envelope protein; at least one second viral vector, the second viral vector carries a second nucleic acid molecule, the second nucleic acid molecule encodes at least one fusion protein, the fusion protein includes at least one single chain antibody and the C-terminal domain of the envelope protein, the single chain antibody is capable of binding to CD28 or CD3, the C-terminal domain of the envelope protein includes a transmembrane region and a intracellular region of the envelope protein, the C-terminal of the at least one single chain antibody is connected to the N-terminal of the C-terminal domain of the envelope protein; the first nucleic acid molecule and the second nucleic acid molecule are arranged to express the envelope protein and the fusion protein, and the envelope protein and the fusion protein are in a non-fusion form. After the viral vector according to the embodiment of the present invention is introduced into the recipient cells, a virus with high virus titer can be packaged, and the single chain antibodies expressed by the viruses, mediated by binding to CD28 or CD3, realize the specific targeted binding of the virus to the immune cell, and then realize the specific infection of immune T cells by the virus, and the packaged virus of the present invention can directly transduce non-preactivated or activated T cells in vitro and ex vivo.

According to the embodiment of the present invention, the viral vectors may further comprise at least one of the following additional technical features:

According to the embodiments of the present invention, the viral vectors are retroviral vectors, lentiviral vectors or other enveloped viral vectors.

According to the embodiments of the present invention, the enveloped virus comprises at least one selected from the group consisting of: Bornaviridae, Nyamaviridae, Arenaviridae, Filoviridae, Hantaviridae, Nairoviridae, Orthomyxoviridae, Paramyxoviridae, Bunyaviridae, Phenuiviridae, Rhabdoviridae, Arteriviridae, Coronaviridae, Flaviviridae, Togaviridae, Hepadnaviridae, Spumavirus, Iridoviridae, Herpesviridae, Poxviridae, and Deltavirus.

According to the embodiments of the present invention, the envelope protein is an envelope G glycoprotein (VSV-G) or a mutant variant thereof from a vesicular stomatitis virus belonging to the family Rhabdoviridae. The envelope G glycoprotein of vesicular stomatitis virus has cell membrane attachment and fusion capabilities. Therefore, the virus packaged by the viral vectors provided herein has cell attachment and infection capabilities.

According to the embodiments of the present invention, the envelope G glycoprotein (VSV-G) has an amino acid sequence shown in SEQ ID NO: 1.

KFTIVFPHNQKGNWKNVPSNYHYCPSSSDLNWHNDLIGTALQVKMPKSHK

AIQADGWMCHASKWVTTCDFRWYGPKYITHSIRSFTPSVEQCKESIEQTKQGTWLNPGF

PPQSCGYATVTDAEAVIVQVTPHHVLVDEYTGEWVDSQFINGKCSNYICPTVHNSTTWHS

DYKVKGLCDSNLISMDITFFSEDGELSSLGKEGTGFRSNYFAYETGGKACKMQYCKHWG

VRLPSGVWFEMADKDLFAAARFPECPEGSSISAPSQTSVDVSLIQDVERILDYSLCQETWS

KIRAGLPISPVDLSYLAPKNPGTGPAFTIINGTLKYFETRYIRVDIAAPILSRMVGMISGTTT

ERELWDDWAPYEDVEIGPNGVLRTSSGYKFPLYMIGHGMLDSDLHLSSKAQVFEHPHIQ

DAASQLPDDESLFFGDTGLSKNPIELVEGWFSSWKSSIASFFFIIGLIIGLFLVLRVGIHLCIK

LKHTKKRQIYTDIEMNRLGK (SEQ ID NO: 1).

According to the embodiments of the present invention, the mutant of the envelope protein has a mutation that weakens the attachment capacity. According to specific embodiments of the present invention, the cell membrane attachment ability of the envelope protein mutant is weakened, which weakens the non-specific cell attachment ability of the packaged virus, but does not affect its membrane fusion ability, and the virus still has the ability to infect cells.

According to the embodiments of the present invention, the mutant of the envelope G glycoprotein has K47Q and R354Q mutations. The cell membrane attachment ability of envelope G glycoprotein mutant with K47Q and R354Q mutations is weakened, but its membrane fusion ability is not affected. Therefore, the envelope G glycoprotein mutant with K47Q and R354Q mutations weakens the non-specific cell attachment ability of the virus obtained by packaging, but it does not affect the ability of the virus to infects cells.

According to the embodiments of the present invention, the mutant of the envelope G glycoprotein has an amino acid sequence shown in SEQ ID NO: 2.

KFTIVFPHNQKGNWKNVPSNYHYCPSSSDLNWHNDLIGTALQVKMPQSHK

AIQADGWMCHASKWVTTCDFRWYGPKYITHSIRSFTPSVEQCKESIEQTKQGTWLNPGF

PPQSCGYATVTDAEAVIVQVTPHHVLVDEYTGEWVDSQFINGKCSNYICPTVHNSTTWHS

DYKVKGLCDSNLISMDITFFSEDGELSSLGKEGTGFRSNYFAYETGGKACKMQYCKHWG

VRLPSGVWFEMADKDLFAAARFPECPEGSSISAPSQTSVDVSLIQDVERILDYSLCQETWS

KIRAGLPISPVDLSYLAPKNPGTGPAFTIINGTLKYFETRYIRVDIAAPILSRMVGMISGTTT

EQELWDDWAPYEDVEIGPNGVLRTSSGYKFPLYMIGHGMLDSDLHLSSKAQVFEHPHIQ

DAASQLPDDESLFFGDTGLSKNPIELVEGWFSSWKSSIASFFFIIGLIIGLFLVLRVGIHLCIK

LKHTKKRQIYTDIEMNRLGK (SEQ ID NO: 2).

According to the embodiments of the present invention, the single chain antibody is capable of binding to CD28. According to the embodiments of the present invention, the single chain antibody can specifically target and bind to CD28 and is an anti-CD28 single chain antibody.
According to the embodiments of the present invention, the single chain antibody has an amino acid sequence shown in SEQ ID NO: 3 or 4. The single chain antibodies according to embodiments of the present invention can target and bind to CD28 positive cells, such as T cells.

QVQLVQSGAEVKKPGASVKVSCKASGYTFTSYYIHWVRQAPGQGLEWIGCI

YPGNVNTNYNEKFKDRATLTVDTSISTAYMELSRLRSDDTAVYFCTRSHYGLDWNFDVW

GQGTTVTVSSGGGGSGGGGSGGGGSDIQMTQSPSSLSASVGDRVTITCHASQNIYVWLN

WYQQKPGKAPKLLIYKASNLHTGVPSRFSGSGSGTDFTLTISSLQPEDFATYYCQQGQTY

PYTFGGGTKVEIKE (SEQ ID NO: 3).

DIELTQSPASLAVSLGQRATISCRASESVEYYVTSLMQWYQQKPGQPPKLLIF

AASNVESGVPARFSGSGSGTNFSLNIHPVDEDDVAMYFCQQSRKVPYTFGGGTKLEIKRG

GGGSGGGGSGGGGSQVQLQESGPGLVTPSQSLSITCTVSGFSLSDYGVHWVRQSPGQGL

EWLGVIWAGGGTNYNSALMSRKSISKDNSKSQVFLKMNSLQADDTAVYYCARDKGYSY

YYSMDYWGQGTTVTVSS (SEQ ID NO: 4).

According to the embodiments of the present invention, the single chain antibody is capable of binding to CD3. According to the embodiments of the present invention, the single chain antibody can specifically target and bind to CD3 and is an anti-CD3 single chain antibody.
According to the embodiments of the present invention, the single chain antibody has an amino acid sequence shown in SEQ ID NO: 5 or 6. The single chain antibodies according to embodiments of the present invention can target and bind to CD3 positive cells, such as T cells.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELK (SEQ ID NO: 5).

QVQLQQSGAELARPGASVKMSCKASGYTFTRYTMHWVKQRPGQGLEWIG YINPSRGYTNYNQKFKDKATLTTDKSSSTAYMQLSSLTSEDSAVYYCARYYDDHYCLDY WGQGTTLTVSSGGGGSGGGGSGGGGSQIVLTQSPAIMSASPGEKVTMTCSASSSVSYMN WYQQKSGTSPKRWIYDTSKLASGVPAHFRGSGSGTSYSLTISGMEAEDAATYYCQQWSS NPFTFGSGTKLEINR (SEQ ID NO: 6).

According to the embodiments of the present invention, the fusion protein includes a first single chain antibody, a second single chain antibody and a C-terminal domain of the envelope protein. The first single chain antibody is capable of binding to CD28, and the second single chain antibody is capable of binding to CD3. The C-terminal of the first single chain antibody is connected to the N-terminal of the second single chain antibody, and the C-terminal of the second single chain antibody is connected to the N-terminal of the C-terminal domain of the envelope protein; or, the C-terminal of the second single chain antibody is connected to the N-terminal of the first single chain antibody, and the C-terminal of the first single chain antibody is connected to the N-terminal of the C-terminal domain of the envelope protein. The fusion proteins according to embodiments of the present invention can specifically target and bind to CD28 and CD3. The fusion proteins according to embodiments of the present invention can specifically target and bind to CD28 and CD3 positive cells, such as T cells.

According to the embodiments of the present invention, the first single chain antibody has an amino acid sequence shown in SEQ ID NO: 3 or 4.

According to the embodiments of the present invention, the second single chain antibody has an amino acid sequence shown in SEQ ID NO: 5 or 6.

According to the embodiments of the present invention, the C-terminal domain of the envelope protein further includes at least a portion of the extracellular region of the envelope protein.

According to the embodiments of the present invention, the fusion protein further includes a first linking peptide, wherein the N-terminal of the first linking peptide is connected to the C-terminal of the first single chain antibody, and the C-terminal of the first linking peptide is connected to the N-terminal of the second single chain antibody; or, the N-terminal of the first linking peptide is connected to the C-terminal of the second single chain antibody, and the C-terminal of the first linking peptide is connected to the N-terminal of the first single chain antibody.

According to the embodiments of the present invention, the first linking peptide has any one of an amino acid sequence shown in SEQ ID NO: 7-11.

GGGGSGGGGSGGGGSGGGGSGGGGS (SEQ ID NO: 7).
GGGGSGGGGSGGGGSGGGGS (SEQ ID NO: 8).
GGGGSGGGGSGGGGS (SEQ ID NO: 9).
EAAAKEAAAKEAAAKE (SEQ ID NO: 10).
EAAAKEAAAKEAAAKEAAAK (SEQ ID NO: 11).

According to the embodiments of the present invention, the fusion protein further comprises a second linking peptide, the N-terminal of the second linking peptide is connected to the C-terminal of the at least one single chain antibody, and the C-terminal of the second linking peptide is connected to the N-terminal of the C-terminal domain of the envelope protein.

According to the embodiments of the present invention, the second linking peptide has an amino acid sequence shown in SEQ ID NO: 12. This further separates the single chain antibody region from the C-terminal region of the envelope protein to reduce functional interference between the two.

AAATTT (SEQ ID NO: 12).

According to the embodiments of the present invention, the C-terminal domain of the envelope protein comprises a peptide chain between the 386th amino acid ~ the 434th amino acid and the 495th amino acid of the envelope protein.

According to the embodiments of the present invention, the first amino acid of the N-terminal of the envelope protein is used as the first amino acid, for example, with reference to the envelope G glycoprotein having the amino acid sequence shown in SEQ ID NO: 1 or 2, the C-terminal domain of the envelope protein comprises the peptide chain between the 386th amino acid ~ the 434th amino acid and the 495th amino acid (the first amino acid at the C-terminal) of the envelope protein, that is, if the length of the C-terminal domain of the envelope protein is longer than 61 amino acids and shorter than 111 amino acids (that is, not shorter than 62 amino acids and not longer than 110 amino acids), the packaged lentiviral vector has the highest transduction efficiency.

According to the embodiments of the present invention, the C-terminal domain of the envelope protein comprises a peptide chain, the peptide chain starts from an amino acid between the 395th and the 425th amino acid, to the 495th amino acid of the envelope protein.

According to the embodiments of the present invention, the C-terminal domain of the envelope protein comprises the 425-495th amino acid, the 415-495th amino acid, the 405-495th amino acid, or the 395-495th amino acid of the VSV-G protein.

According to the embodiments of the present invention, the C-terminal domain of the envelope protein has an amino acid sequence shown in SEQ ID NO: 13, 39, 40 or 41.

FEHPHIQDAASQLPDDESLFFGDTGLSKNPIELVEGWFSSWKSSIASFFFIIGLI

IGLFLVLRVGIHLCIKLKHTKKRQIYTDIEMNRLGK (SEQ ID NO: 13).

FGDTGLSKNPIELVEGWFSSWKSSIASFFFIIGLIIGLFLVLRVGIHLCIKLKHTK

KRQIYTDIEMNRLGK (SEQ ID NO: 39).

SQLPDDESLFFGDTGLSKNPIELVEGWFSSWKSSIASFFFIIGLIIGLFLVLRVGI

HLCIKLKHTKKRQIYTDIEMNRLGK (SEQ ID NO: 40).

DLHLSSKAQVFEHPHIQDAASQLPDDESLFFGDTGLSKNPIELVEGWFSSWK

SSIASFFFIIGLIIGLFLVLRVGIHLCIKLKHTKKRQIYTDIEMNRLGK (SEQ ID NO: 41).

According to the embodiments of the present invention, the fusion protein has an amino acid sequence shown in SEQ ID NO:14, 15, 16, 17, 18, 19 or 20.

QVQLVQSGAEVKKPGASVKVSCKASGYTFTSYYIHWVRQAPGQGLEWIGCI

YPGNVNTNYNEKFKDRATLTVDTSISTAYMELSRLRSDDTAVYFCTRSHYGLDWNFDVW

GQGTTVTVSSGGGGSGGGGSGGGGSDIQMTQSPSSLSASVGDRVTITCHASQNIYVWLN

WYQQKPGKAPKLLIYKASNLHTGVPSRFSGSGSGTDFTLTISSLQPEDFATYYCQQGQTY

PYTFGGGTKVEIKEAAATTTFEHPHIQDAASQLPDDESLFFGDTGLSKNPIELVEGWFSSW

KSSIASFFFIIGLIIGLFLVLRVGIHLCIKLKHTKKRQIYTDIEMNRLGK (SEQ ID NO: 14).

DIELTQSPASLAVSLGQRATISCRASESVEYYVTSLMQWYQQKPGQPPKLLIF

AASNVESGVPARFSGSGSGTNFSLNIHPVDEDDVAMYFCQQSRKVPYTFGGGTKLEIKRG

GGGSGGGGSGGGGSQVQLQESGPGLVTPSQSLSITCTVSGFSLSDYGVHWVRQSPGQGL EWLGVIWAGGGTNYNSALMSRKSISKDNSKSQVFLKMNSLQADDTAVYYCARDKGYSY YYSMDYWGQGTTVTVSSAAATTTFEHPHIQDAASQLPDDESLFFGDTGLSKNPIELVEG WFSSWKSSIASFFFIIGLIIGLFLVLRVGIHLCIKLKHTKKRQIYTDIEMNRLGK (SEQ ID NO: 18).

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTFEHPHIQDAASQLPDDESLFFGDTGLSKNPIELVEGWFSS WKSSIASFFFIIGLIIGLFLVLRVGIHLCIKLKHTKKRQIYTDIEMNRLGK (SEQ ID NO: 15).

QVQLQQSGAELARPGASVKMSCKASGYTFTRYTMHWVKQRPGQGLEWIG YINPSRGYTNYNQKFKDKATLTTDKSSSTAYMQLSSLTSEDSAVYYCARYYDDHYCLDY WGQGTTLTVSSGGGGSGGGGSGGGGSQIVLTQSPAIMSASPGEKVTMTCSASSSVSYMN WYQQKSGTSPKRWIYDTSKLASGVPAHFRGSGSGTSYSLTISGMEAEDAATYYCQQWSS NPFTFGSGTKLEINRAAATTTFEHPHIQDAASQLPDDESLFFGDTGLSKNPIELVEGWFSS WKSSIASFFFIIGLIIGLFLVLRVGIHLCIKLKHTKKRQIYTDIEMNRLGK (SEQ ID NO: 19).

QVQLVQSGAEVKKPGASVKVSCKASGYTFTSYYIHWVRQAPGQGLEWIGCI YPGNVNTNYNEKFKDRATLTVDTSISTAYMELSRLRSDDTAVYFCTRSHYGLDWNFDVW GQGTTVTVSSGGGGSGGGGSGGGGSDIQMTQSPSSLSASVGDRVTITCHASQNIYVWLN WYQQKPGKAPKLLIYKASNLHTGVPSRFSGSGSGTDFTLTISSLQPEDFATYYCQQGQTY PYTFGGGTKVEIKEGGGGSGGGGSGGGGSGGGGSGGGGSDIKLQQSGAELARPGASVK MSCKTSGYTFTRYTMHWVKQRPGQGLEWIGYINPSRGYTNYNQKFKDKATLTTDKSSST AYMQLSSLTSEDSAVYYCARYYDDHYCLDYWGQGTTLTVSSVEGGSGGSGGSGGSGGV DDIQLTQSPAIMSASPGEKVTMTCRASSSVSYMNWYQQKSGTSPKRWIYDTSKVASGVP YRFSGSGSGTSYSLTISSMEAEDAATYYCQQWSSNPLTFGAGTKLELKAAATTTFEHPHIQ DAASQLPDDESLFFGDTGLSKNPIELVEGWFSSWKSSIASFFFIIGLIIGLFLVLRVGIHLCIK LKHTKKRQIYTDIEMNRLGK (SEQ ID NO: 16).

DIELTQSPASLAVSLGQRATISCRASESVEYYVTSLMQWYQQKPGQPPKLLIF

AASNVESGVPARFSGSGSGTNFSLNIHPVDEDDVAMYFCQQSRKVPYTFGGGTKLEIKRG

GGGSGGGGSGGGGSQVQLQESGPGLVTPSQSLSITCTVSGFSLSDYGVHWVRQSPGQGL

EWLGVIWAGGGTNYNSALMSRKSISKDNSKSQVFLKMNSLQADDTAVYYCARDKGYSY

YYSMDYWGQGTTVTVSSGGGGSGGGGSGGGGSGGGGSGSGGGGSGGGGSGGGGSQV

QLQQSGAELARPGASVKMSCKASGYTFTRYTMHWVKQRPGQGLEWIGYINPSRGYTNY

NQKFKDKATLTTDKSSSTAYMQLSSLTSEDSAVYYCARYYDDHYCLDYWGQGTTLTVSS

GGGGSGGGGSGGGGSQIVLTQSPAIMSASPGEKVTMTCSASSSVSYMNWYQQKSGTSPK

RWIYDTSKLASGVPAHFRGSGSGTSYSLTISGMEAEDAATYYCQQWSSNPFTFGSGTKLEI

NRAAATTTFEHPHIQDAASQLPDDESLFFGDTGLSKNPIELVEGWFSSWKSSIASFFFIIGLI

IGLFLVLRVGIHLCIKLKHTKKRQIYTDIEMNRLGK (SEQ ID NO: 20).

DIKLQQSGAELARPGASVKMSCKTSGYTFTRYTMHWVKQRPGQGLEWIGY

INPSRGYTNYNQKFKDKATLTTDKSSSTAYMQLSSLTSEDSAVYYCARYYDDHYCLDYW

GQGTTLTVSSVEGGSGGSGGSGGSGGVDDIQLTQSPAIMSASPGEKVTMTCRASSSVSYM

NWYQQKSGTSPKRWIYDTSKVASGVPYRFSGSGSGTSYSLTISSMEAEDAATYYCQQWS

SNPLTFGAGTKLELKGGGGSGGGGSGGGGSGGGGSGGGGSQVQLVQSGAEVKKPGASV

KVSCKASGYTFTSYYIHWVRQAPGQGLEWIGCIYPGNVNTNYNEKFKDRATLTVDTSIST

AYMELSRLRSDDTAVYFCTRSHYGLDWNFDVWGQGTTVTVSSGGGGSGGGGSGGGGS

DIQMTQSPSSLSASVGDRVTITCHASQNIYVWLNWYQQKPGKAPKLLIYKASNLHTGVPS

RFSGSGSGTDFTLTISSLQPEDFATYYCQQGQTYPYTFGGGTKVEIKEAAATTTFEHPHIQD

AASQLPDDESLFFGDTGLSKNPIELVEGWFSSWKSSIASFFFIIGLIIGLFLVLRVGIHLCIKL

KHTKKRQIYTDIEMNRLGK (SEQ ID NO: 17).

Wherein, the fusion protein having the amino acid sequence shown in SEQ ID NO: 14 is a fusion protein comprising a single chain antibody capable of binding to CD28, in this application, the code for expressing the fusion protein having the amino acid sequence shown in SEQ ID NO: 14 is: S1.

The fusion protein having the amino acid sequence shown in SEQ ID NO: 18 is a fusion protein comprising a single chain antibody capable of binding to CD28, in this application, the code for expressing the fusion protein having the amino acid sequence shown in SEQ ID NO: 18 is: S3.

The fusion protein having the amino acid sequence shown in SEQ ID NO: 15 is a fusion protein comprising a single chain antibody capable of binding to CD3, in this application, the code for expressing the fusion protein having the amino acid sequence shown in SEQ ID NO: 15 is: S2.

The fusion protein having the amino acid sequence shown in SEQ ID NO: 19 is a fusion protein comprising a single chain antibody capable of binding to CD3, in this application, the code for expressing the fusion protein having the amino acid sequence shown in SEQ ID NO: 19 is: S4.

The fusion protein having the amino acid sequence shown in SEQ ID NO: 16 is a fusion protein comprising a single chain antibody capable of binding to CD28 and CD3, in this application, the code for expressing the fusion protein having the amino acid sequence shown in SEQ ID NO: 16 is: S 12.

The fusion protein having the amino acid sequence shown in SEQ ID NO: 20 is a fusion protein comprising a single chain antibody capable of binding to CD28 and CD3, in this application, the code for expressing the fusion protein having the amino acid sequence shown in SEQ ID NO: 20 is: S34.

The fusion protein having the amino acid sequence shown in SEQ ID NO: 17 is a fusion protein comprising a single chain antibody capable of binding to CD28 and CD3.

According to the embodiments of the present invention, the viral vector further comprises: a first promoter, which is operably linked to the first nucleic acid molecule; and a second promoter, which is operably linked to the second nucleic acid molecule. Thereby, the first nucleic acid molecule and the second nucleic acid molecule are respectively under the regulation of the first promoter and the second promoter to achieve high-efficiency expression of the first nucleic acid molecule and the second nucleic acid molecule.

According to the embodiments of the present invention, each of the first promoter and the second promoter is independently selected from CMV, EF-1 or RSV promoters.

According to the embodiments of the present invention, the first nucleic acid molecule has a nucleotide sequence shown in SEQ ID NO: 21 or 35.

AAGTTCACCATAGTTTTTCCACACAACCAAAAAGGAAACTGGAAAAATG

TTCCTTCTAATTACCATTATTGCCCGTCAAGCTCAGATTTAAATTGGCATAATGACTTAA

TAGGCACAGCCTTACAAGTCAAAATGCCCCAAAGTCACAAGGCTATTCAAGCAGACG

GTTGGATGTGTCATGCTTCCAAATGGGTCACTACTTGTGATTTCCGCTGGTATGGACCG

AAGTATATAACACATTCCATCCGATCCTTCACTCCATCTGTAGAACAATGCAAGGAAAG

CATTGAACAAACGAAACAAGGAACTTGGCTGAATCCAGGCTTCCCTCCTCAAAGTTG

TGGATATGCAACTGTGACGGATGCCGAAGCAGTGATTGTCCAGGTGACTCCTCACCAT

GTGCTGGTTGATGAATACACAGGAGAATGGGTTGATTCACAGTTCATCAACGGAAAT

GCAGCAATTACATATGCCCCACTGTCCATAACTCTACAACCTGGCATTCTGACTATAAG

GTCAAAGGGCTATGTGATTCTAACCTCATTTCCATGGACATCACCTTCTTCTCAGAGGA

CGGAGAGCTATCATCCCTGGGAAAGGAGGGCACAGGGTTCAGAAGTAACTACTTTGC

TTATGAAACTGGAGGCAAGGCCTGCAAAATGCAATACTGCAAGCATTGGGGAGTCAG

ACTCCCATCAGGTGTCTGGTTCGAGATGGCTGATAAGGATCTCTTTGCTGCAGCCAGA

TTCCCTGAATGCCCAGAAGGGTCAAGTATCTCTGCTCCATCTCAGACCTCAGTGGATG

TAAGTCTAATTCAGGACGTTGAGAGGATCTTGGATTATTCCCTCTGCCAAGAAACCTG

GAGCAAAATCAGAGCGGGTCTTCCAATCTCTCCAGTGGATCTCAGCTATCTTGCTCCT

AAAAACCCAGGAACCGGTCCTGCTTTCACCATAATCAATGGTACCCTAAAATACTTTG

AGACCAGATACATCAGAGTCGATATTGCTGCTCCAATCCTCTCAAGAATGGTCGGAAT

GATCAGTGGAACTACCACAGAACAGGAACTGTGGGATGACTGGGCACCATATGAAGA

CGTGGAAATTGGACCCAATGGAGTTCTGAGGACCAGTTCAGGATATAAGTTTCCTTTAT

ACATGATTGGACATGGTATGTTGGACTCCGATCTTCATCTTAGCTCAAAGGCTCAGGTG

TTCGAACATCCTCACATTCAAGACGCTGCTTCGCAACTTCCTGATGATGAGAGTTTATT

TTTTGGTGATACTGGGCTATCCAAAAATCCAATCGAGCTTGTAGAAGGTTGGTTCAGT

AGTTGGAAAAGCTCTATTGCCTCTTTTTTCTTTATCATAGGGTTAATCATTGGACTATTC

TTGGTTCTCCGAGTTGGTATCCATCTTTGCATTAAATTAAAGCACACCAAGAAAAGAC

AGATTTATACAGACATAGAGATGAACCGACTTGGAAAG (SEQ ID NO: 21).

AAGTTCACCATAGTTTTTCCACACAACCAAAAAGGAAACTGGAAAAATGTTCCTTCTAATTACCATTATTGCCCGTCAAGCTCAGATTTAAATTGGCATAATGACTTAATAGGCACAGCCTTACAAGTCAAAATGCCCAAGAGTCACAAGGCTATTCAAGCAGACGGTTGGATGTGTCATGCTTCCAAATGGGTCACTACTTGTGATTTCCGCTGGTATGGACCGAAGTATATAACACATTCCATCCGATCCTTCACTCCATCTGTAGAACAATGCAAGGAAAGCATTGAACAAACGAAACAAGGAACTTGGCTGAATCCAGGCTTCCCTCCTCAAAGTTGTGGATATGCAACTGTGACGGATGCCGAAGCAGTGATTGTCCAGGTGACTCCTCACCATGTGCTGGTTGATGAATACACAGGAGAATGGGTTGATTCACAGTTCATCAACGGAAATGCAGCAATTACATATGCCCCACTGTCCATAACTCTACAACCTGGCATTCTGACTATAAGGTCAAAGGGCTATGTGATTCTAACCTCATTTCCATGGACATCACCTTCTTCTCAGAGGACGGAGAGCTATCATCCCTGGGAAAGGAGGGCACAGGGTTCAGAAGTAACTACTTTGCTTATGAAACTGGAGGCAAGGCCTGCAAAATGCAATACTGCAAGCATTGGGGAGTCAGACTCCCATCAGGTGTCTGGTTCGAGATGGCTGATAAGGATCTCTTTGCTGCAGCCAGATTCCCTGAATGCCCAGAAGGGTCAAGTATCTCTGCTCCATCTCAGACCTCAGTGGATGTAAGTCTAATTCAGGACGTTGAGAGGATCTTGGATTATTCCCTCTGCCAAGAAACCTGGAGCAAAATCAGAGCGGGTCTTCCAATCTCTCCAGTGGATCTCAGCTATCTTGCTCCTAAAAACCCAGGAACCGGTCCTGCTTTCACCATAATCAATGGTACCCTAAAATACTTTGAGACCAGATACATCAGAGTCGATATTGCTGCTCCAATCCTCTCAAGAATGGTCGGAATGATCAGTGGAACTACCACAGAAAGGGAACTGTGGGATGACTGGGCACCATATGAAGACGTGGAAATTGGACCCAATGGAGTTCTGAGGACCAGTTCAGGATATAAGTTTCCTTTATACATGATTGGACATGGTATGTTGGACTCCGATCTTCATCTTAGCTCAAAGGCTCAGGTGTTCGAACATCCTCACATTCAAGACGCTGCTTCGCAACTTCCTGATGATGAGAGTTTATTTTTTGGTGATACTGGGCTATCCAAAAATCCAATCGAGCTTGTAGAAGGTTGGTTCAGTAGTTGGAAAGCTCTATTGCCTCTTTTTTCTTTATCATAGGGTTAATCATTGGACTATTCTTGGTTCTCCGAGTTGGTATCCATCTTTGCATTAAATTAAAGCACACCAAGAAAGACAGATTTATACAGACATAGAGATGAACCGACTTGGAAAG (SEQ ID NO: 35).

According to the embodiments of the present invention, the second nucleic acid molecule has a nucleotide sequence shown in SEQ ID NO: 22, 23, 24, 25, 32, 33 or 34.

Wherein, the fusion protein encoded by the second nucleic acid molecule having the nucleotide sequence shown in SEQ ID NO:22 is a fusion protein comprising a single chain antibody capable of binding to CD28, in this application, the code of the fusion protein encoded by the nucleotide sequence shown in SEQ ID NO:22 is: S1.

The fusion protein encoded by the second nucleic acid molecule having the nucleotide sequence shown in SEQ ID NO:23 is a fusion protein comprising a single chain antibody capable of binding to CD3, in this application, the code of the fusion protein encoded by the nucleotide sequence shown in SEQ ID NO:23 is: S2.

The fusion protein encoded by the second nucleic acid molecule having the nucleotide sequence shown in SEQ ID NO:24 is a fusion protein comprising a single chain antibody capable of binding to CD28 and CD3, in this application, the code of the fusion protein encoded by the nucleotide sequence shown in SEQ ID NO:24 is: S 12.

The fusion protein encoded by the second nucleic acid molecule having the nucleotide sequence shown in SEQ ID NO:25 is a fusion protein comprising a single chain antibody capable of binding to CD3 and CD28. In this application, the nucleotide sequence shown in SEQ ID NO:25 encodes a fusion protein having the amino acid sequence shown in SEQ ID NO: 17.

The fusion protein having the nucleotide sequence shown in SEQ ID NO: 32 is a fusion protein comprising a single chain antibody capable of binding to CD28, in this application, the code of the fusion protein encoded by the nucleotide sequence shown in SEQ ID NO:32 is: S3.

The fusion protein having the nucleotide sequence shown in SEQ ID NO: 33 is a fusion protein comprising a single chain antibody capable of binding to CD3, in this application, the code of the fusion protein encoded by the nucleotide sequence shown in SEQ ID NO:33 is: S4.

The fusion protein having the nucleotide sequence shown in SEQ ID NO: 34 is a fusion protein comprising a single chain antibody capable of binding to CD28 and CD3, in this application, the code of the fusion protein encoded by the nucleotide sequence shown in SEQ ID NO:34 is: S34.

CAGGTGCAGCTGGTGCAGTCTGGGGCTGAGGTGAAGAAGCCTGGGGCCT
CAGTGAAGGTCTCCTGCAAGGCTTCTGGATACACCTTCACCAGCTACTATATACACTG
GGTGCGACAGGCCCCTGGACAAGGGCTTGAGTGGATTGGATGTATTTATCCTGGAAAT
GTCAATACTAACTATAATGAGAAGTTCAAGGACAGGGCCACCCTGACCGTAGACACGT
CCATCAGCACAGCCTACATGGAGCTGAGCAGGCTGAGATCTGACGACACGGCCGTGT
ATTTCTGTACAAGATCACACTACGGCCTCGACTGGAACTTCGATGTCTGGGGCCAAGG
GACCACGGTCACCGTCTCCTCAGGTGGAGGCGGTTCAGGCGGAGGTGGCAGCGGCG
GTGGCGGGTCGGACATCCAGATGACCCAGTCTCCATCCTCCCTGTCTGCATCTGTAGG
AGACAGAGTCACCATCACTTGCCATGCCAGTCAAAACATTTATGTTTGGTTAAACTGG
TATCAGCAGAAACCAGGGAAAGCCCCTAAGCTCCTGATCTATAAGGCTTCCAACCTGC
ACACAGGGGTCCCATCAAGGTTCAGTGGCAGTGGATCTGGGACAGATTTCACTCTCA
CCATCAGCAGTCTGCAACCTGAAGATTTTGCAACTTACTACTGTCAACAGGGTCAAAC
TTATCCGTACACGTTCGGCGGAGGGACCAAGGTGGAGATCAAAGAGGCGGCCGCAAC
TACCACCTTCGAACATCCTCACATTCAAGACGCTGCTTCGCAACTTCCTGATGATGAG
AGTTTATTTTTTGGTGATACTGGGCTATCCAAAAATCCAATCGAGCTTGTAGAAGGTTG
GTTCAGTAGTTGGAAAAGCTCTATTGCCTCTTTTTTCTTTATCATAGGGTTAATCATTGG
ACTATTCTTGGTTCTCCGAGTTGGTATCCATCTTTGCATTAAATTAAAGCACACCAAGA
AAAGACAGATTTATACAGACATAGAGATGAACCGACTTGGAAAG (SEQ ID NO: 22).

GATATCAAACTGCAGCAGTCAGGGGCTGAACTGGCAAGACCTGGGGCCT
CAGTGAAGATGTCCTGCAAGACTTCTGGCTACACCTTTACTAGGTACACGATGCACTG
GGTAAAACAGAGGCCTGGACAGGGTCTGGAATGGATTGGATACATTAATCCTAGCCGT
GGTTATACTAATTACAATCAGAAGTTCAAGGACAAGGCCACATTGACTACAGACAAAT
CCTCCAGCACAGCCTACATGCAACTGAGCAGCCTGACATCTGAGGACTCTGCAGTCTA
TTACTGTGCAAGATATTATGATGATCATTACTGCCTTGACTACTGGGGCCAAGGCACCA
CTCTCACAGTCTCCTCAGTCGAAGGTGGAAGTGGAGGTTCTGGTGGAAGTGGAGGTT
CAGGTGGAGTCGACGACATTCAGCTGACCCAGTCTCCAGCAATCATGTCTGCATCTCC
AGGGGAGAAGGTCACCATGACCTGCAGAGCCAGTTCAAGTGTAAGTTACATGAACTG
GTACCAGCAGAAGTCAGGCACCTCCCCCAAAAGATGGATTTATGACACATCCAAAGT
GGCTTCTGGAGTCCCTTATCGCTTCAGTGGCAGTGGGTCTGGGACCTCATACTCTCTC
ACAATCAGCAGCATGGAGGCTGAAGATGCTGCCACTTATTACTGCCAACAGTGGAGTA
GTAACCCGCTCACGTTCGGTGCTGGGACCAAGCTGGAGCTGAAAGCGGCCGCAACTA
CCACCTTCGAACATCCTCACATTCAAGACGCTGCTTCGCAACTTCCTGATGATGAGAG
TTTATTTTTGGTGATACTGGGCTATCCAAAAATCCAATCGAGCTTGTAGAAGGTTGGT
TCAGTAGTTGGAAAAGCTCTATTGCCTCTTTTTTCTTTATCATAGGGTTAATCATTGGAC
TATTCTTGGTTCTCCGAGTTGGTATCCATCTTTGCATTAAATTAAAGCACACCAAGAAA
AGACAGATTTATACAGACATAGAGATGAACCGACTTGGAAAG (SEQ ID NO: 23).

CAGGTGCAGCTGGTGCAGTCTGGGGCTGAGGTGAAGAAGCCTGGGGCCT
CAGTGAAGGTCTCCTGCAAGGCTTCTGGATACACCTTCACCAGCTACTATATACACTG
GGTGCGACAGGCCCCTGGACAAGGGCTTGAGTGGATTGGATGTATTTATCCTGGAAAT
GTCAATACTAACTATAATGAGAAGTTCAAGGACAGGGCCACCCTGACCGTAGACACGT
CCATCAGCACAGCCTACATGGAGCTGAGCAGGCTGAGATCTGACGACACGGCCGTGT

ATTTCTGTACAAGATCACACTACGGCCTCGACTGGAACTTCGATGTCTGGGGCCAAGG

GACCACGGTCACCGTCTCCTCAGGTGGAGGCGGTTCAGGCGGAGGTGGCAGCGGCG

GTGGCGGGTCGGACATCCAGATGACCCAGTCTCCATCCTCCCTGTCTGCATCTGTAGG

AGACAGAGTCACCATCACTTGCCATGCCAGTCAAAACATTTATGTTTGGTTAAACTGG

TATCAGCAGAAACCAGGGAAAGCCCCTAAGCTCCTGATCTATAAGGCTTCCAACCTGC

ACACAGGGGTCCCATCAAGGTTCAGTGGCAGTGGATCTGGGACAGATTTCACTCTCA

CCATCAGCAGTCTGCAACCTGAAGATTTTGCAACTTACTACTGTCAACAGGGTCAAAC

TTATCCGTACACGTTCGGCGGAGGGACCAAGGTGGAGATCAAAGAGGGAGGCGGCG

GCAGCGGCGGGGGAGGGTCCGGAGGGGGTGGTTCTGGTGGAGGAGGATCGGGAGGC

GGTGGCAGCGATATCAAACTGCAGCAGTCAGGGGCTGAACTGGCAAGACCTGGGGCC

TCAGTGAAGATGTCCTGCAAGACTTCTGGCTACACCTTTACTAGGTACACGATGCACT

GGGTAAAACAGAGGCCTGGACAGGGTCTGGAATGGATTGGATACATTAATCCTAGCCG

TGGTTATACTAATTACAATCAGAAGTTCAAGGACAAGGCCACATTGACTACAGACAAA

TCCTCCAGCACAGCCTACATGCAACTGAGCAGCCTGACATCTGAGGACTCTGCAGTCT

ATTACTGTGCAAGATATTATGATGATCATTACTGCCTTGACTACTGGGGCCAAGGCACC

ACTCTCACAGTCTCCTCAGTCGAAGGTGGAAGTGGAGGTTCTGGTGGAAGTGGAGGT

TCAGGTGGAGTCGACGACATTCAGCTGACCCAGTCTCCAGCAATCATGTCTGCATCTC

CAGGGGAGAAGGTCACCATGACCTGCAGAGCCAGTTCAAGTGTAAGTTACATGAACT

GGTACCAGCAGAAGTCAGGCACCTCCCCCAAAAGATGGATTTATGACACATCCAAAG

TGGCTTCTGGAGTCCCTTATCGCTTCAGTGGCAGTGGGTCTGGGACCTCATACTCTCTC

ACAATCAGCAGCATGGAGGCTGAAGATGCTGCCACTTATTACTGCCAACAGTGGAGTA

GTAACCCGCTCACGTTCGGTGCTGGGACCAAGCTGGAGCTGAAAGCGGCCGCAACTA

CCACCTTCGAACATCCTCACATTCAAGACGCTGCTTCGCAACTTCCTGATGATGAGAG

TTTATTTTTTGGTGATACTGGGCTATCCAAAAATCCAATCGAGCTTGTAGAAGGTTGGT

TCAGTAGTTGGAAAAGCTCTATTGCCTCTTTTTTCTTTATCATAGGGTTAATCATTGGAC

TATTCTTGGTTCTCCGAGTTGGTATCCATCTTTGCATTAAATTAAAGCACACCAAGAAA

AGACAGATTTATACAGACATAGAGATGAACCGACTTGGAAAG (SEQ ID NO: 24).

GATATCAAACTGCAGCAGTCAGGGGCTGAACTGGCAAGACCTGGGGCCT

CAGTGAAGATGTCCTGCAAGACTTCTGGCTACACCTTTACTAGGTACACGATGCACTG

GGTAAAACAGAGGCCTGGACAGGGTCTGGAATGGATTGGATACATTAATCCTAGCCGT

GGTTATACTAATTACAATCAGAAGTTCAAGGACAAGGCCACATTGACTACAGACAAAT

CCTCCAGCACAGCCTACATGCAACTGAGCAGCCTGACATCTGAGGACTCTGCAGTCTA

TTACTGTGCAAGATATTATGATGATCATTACTGCCTTGACTACTGGGGCCAAGGCACCA

CTCTCACAGTCTCCTCAGTCGAAGGTGGAAGTGGAGGTTCTGGTGGAAGTGGAGGTT

CAGGTGGAGTCGACGACATTCAGCTGACCCAGTCTCCAGCAATCATGTCTGCATCTCC

AGGGGAGAAGGTCACCATGACCTGCAGAGCCAGTTCAAGTGTAAGTTACATGAACTG

GTACCAGCAGAAGTCAGGCACCTCCCCCAAAAGATGGATTTATGACACATCCAAAGT

GGCTTCTGGAGTCCCTTATCGCTTCAGTGGCAGTGGGTCTGGGACCTCATACTCTCTC

ACAATCAGCAGCATGGAGGCTGAAGATGCTGCCACTTATTACTGCCAACAGTGGAGTA

GTAACCCGCTCACGTTCGGTGCTGGGACCAAGCTGGAGCTGAAAGGAGGCGGCGGC

AGCGGCGGGGGAGGGTCCGGAGGGGGTGGTTCTGGTGGAGGAGGATCGGGAGGCGG

TGGCAGCCAGGTGCAGCTGGTGCAGTCTGGGGCTGAGGTGAAGAAGCCTGGGGCCT

CAGTGAAGGTCTCCTGCAAGGCTTCTGGATACACCTTCACCAGCTACTATATACACTG

GGTGCGACAGGCCCCTGGACAAGGGCTTGAGTGGATTGGATGTATTTATCCTGGAAAT

GTCAATACTAACTATAATGAGAAGTTCAAGGACAGGGCCACCCTGACCGTAGACACGT

CCATCAGCACAGCCTACATGGAGCTGAGCAGGCTGAGATCTGACGACACGGCCGTGT

ATTTCTGTACAAGATCACACTACGGCCTCGACTGGAACTTCGATGTCTGGGGCCAAGG

GACCACGGTCACCGTCTCCTCAGGTGGAGGCGGTTCAGGCGGAGGTGGCAGCGGCG

GTGGCGGGTCGGACATCCAGATGACCCAGTCTCCATCCTCCCTGTCTGCATCTGTAGG

AGACAGAGTCACCATCACTTGCCATGCCAGTCAAAACATTTATGTTTGGTTAAACTGG

TATCAGCAGAAACCAGGGAAAGCCCCTAAGCTCCTGATCTATAAGGCTTCCAACCTGC

ACACAGGGGTCCCATCAAGGTTCAGTGGCAGTGGATCTGGGACAGATTTCACTCTCA

CCATCAGCAGTCTGCAACCTGAAGATTTTGCAACTTACTACTGTCAACAGGGTCAAAC

TTATCCGTACACGTTCGGCGGAGGGACCAAGGTGGAGATCAAAGAGGCGGCCGCAAC

TACCACCTTCGAACATCCTCACATTCAAGACGCTGCTTCGCAACTTCCTGATGATGAG

AGTTTATTTTTTGGTGATACTGGGCTATCCAAAAATCCAATCGAGCTTGTAGAAGGTTG

GTTCAGTAGTTGGAAAAGCTCTATTGCCTCTTTTTTCTTTATCATAGGGTTAATCATTGG

ACTATTCTTGGTTCTCCGAGTTGGTATCCATCTTTGCATTAAATTAAAGCACACCAAGA

AAAGACAGATTTATACAGACATAGAGATGAACCGACTTGGAAAG (SEQ ID NO: 25).

GACATCGAGCTCACTCAGTCTCCAGCTTCTTTGGCTGTGTCTCTAGGGCA

GAGAGCCACCATCTCCTGCAGAGCCAGTGAGAGTGTTGAATATTATGTCACAAGTTTA

ATGCAGTGGTACCAGCAGAAGCCAGGACAGCCACCCAAACTCCTCATCTTTGCTGCAT

CCAACGTAGAATCTGGGGTCCCTGCCAGGTTTAGTGGCAGTGGGTCTGGGACAAACT

TCAGCCTCAACATCCATCCTGTGGACGAGGATGATGTTGCAATGTATTTCTGTCAGCAA

AGTAGGAAGGTTCCTTACACGTTCGGAGGGGGGACCAAGCTGGAAATAAAACGAGG

GGGCGGAGGATCCGGTGGAGGCGGAAGCGGGGGTGGAGGATCCCAGGTGCAGCTGC

AGGAGTCAGGACCTGGCCTGGTGACGCCCTCACAGAGCCTGTCCATCACTTGTACTG

TCTCTGGGTTTTCATTAAGCGACTATGGTGTTCATTGGGTTCGCCAGTCTCCAGGACAG

GGACTGGAGTGGCTGGGAGTAATATGGGCTGGTGGAGGCACGAATTATAATTCGGCTC

TCATGTCCAGAAAGAGCATCAGCAAAGACAACTCCAAGAGCCAAGTTTTCTTAAAAA

TGAACAGTCTGCAAGCTGATGACACAGCCGTGTATTACTGTGCCAGAGATAAGGGATA

CTCCTATTACTATTCTATGGACTACTGGGGCCAAGGCACCACGGTCACCGTCTCCTCAG

CGGCCGCAACTACCACCTTCGAACATCCTCACATTCAAGACGCTGCTTCGCAACTTCC

TGATGATGAGAGTTTATTTTTTGGTGATACTGGGCTATCCAAAAATCCAATCGAGCTTG

TAGAAGGTTGGTTCAGTAGTTGGAAAAGCTCTATTGCCTCTTTTTTCTTTATCATAGGG

TTAATCATTGGACTATTCTTGGTTCTCCGAGTTGGTATCCATCTTTGCATTAAATTAAAG

CACACCAAGAAAAGACAGATTTATACAGACATAGAGATGAACCGACTTGGAAAG

(SEQ ID NO: 32).

CAGGTCCAGCTGCAGCAGTCTGGGGCTGAACTGGCAAGACCTGGGGCCT

CAGTGAAGATGTCCTGCAAGGCTTCTGGCTACACCTTTACTAGGTACACGATGCACTG

GGTAAAACAGAGGCCTGGACAGGGTCTGGAATGGATTGGATACATTAATCCTAGCCGT

GGTTATACTAATTACAATCAGAAGTTCAAGGACAAGGCCACATTGACTACAGACAAAT

CCTCCAGCACAGCCTACATGCAACTGAGCAGCCTGACATCTGAGGACTCTGCAGTCTA

TTACTGTGCAAGATATTATGATGATCATTACTGCCTTGACTACTGGGGCCAAGGCACCA

CTCTCACAGTCTCCTCAGGTGGCGGTGGCTCCGGCGGTGGTGGGTCGGGTGGCGGCG

GATCTCAGATTGTGTTGACCCAGTCTCCAGCAATCATGTCTGCATCTCCAGGGGAGAA

GGTTACCATGACCTGCAGTGCCAGCTCAAGTGTAAGTTACATGAACTGGTACCAGCAG

AAGTCAGGCACCTCCCCCAAAAGATGGATTTATGACACATCCAAACTGGCTTCTGGAG

TCCCTGCTCACTTCAGGGGCAGTGGGTCTGGGACCTCTTACTCTCTCACAATCAGCGG

CATGGAGGCTGAAGATGCTGCCACTTATTACTGCCAGCAGTGGAGTAGTAACCCATTC

ACGTTCGGCTCGGGGACCAAGCTGGAGATCAACCGTGCGGCCGCAACTACCACCTTC

GAACATCCTCACATTCAAGACGCTGCTTCGCAACTTCCTGATGATGAGAGTTTATTTTT

TGGTGATACTGGGCTATCCAAAAATCCAATCGAGCTTGTAGAAGGTTGGTTCAGTAGT

TGGAAAAGCTCTATTGCCTCTTTTTTCTTTATCATAGGGTTAATCATTGGACTATTCTTG

GTTCTCCGAGTTGGTATCCATCTTTGCATTAAATTAAAGCACACCAAGAAAAGACAGA

TTTATACAGACATAGAGATGAACCGACTTGGAAAG (SEQ ID NO: 33).

GACATCGAGCTCACTCAGTCTCCAGCTTCTTTGGCTGTGTCTCTAGGGCA

GAGAGCCACCATCTCCTGCAGAGCCAGTGAGAGTGTTGAATATTATGTCACAAGTTTA

ATGCAGTGGTACCAGCAGAAGCCAGGACAGCCACCCAAACTCCTCATCTTTGCTGCAT

CCAACGTAGAATCTGGGGTCCCTGCCAGGTTTAGTGGCAGTGGGTCTGGGACAAACT

TCAGCCTCAACATCCATCCTGTGGACGAGGATGATGTTGCAATGTATTTCTGTCAGCAA

AGTAGGAAGGTTCCTTACACGTTCGGAGGGGGGACCAAGCTGGAAATAAAACGAGG

GGGCGGAGGATCCGGTGGAGGCGGAAGCGGGGGTGGAGGATCCCAGGTGCAGCTGC

AGGAGTCAGGACCTGGCCTGGTGACGCCCTCACAGAGCCTGTCCATCACTTGTACTG

TCTCTGGGTTTTCATTAAGCGACTATGGTGTTCATTGGGTTCGCCAGTCTCCAGGACAG

GGACTGGAGTGGCTGGGAGTAATATGGGCTGGTGGAGGCACGAATTATAATTCGGCTC

TCATGTCCAGAAAGAGCATCAGCAAAGACAACTCCAAGAGCCAAGTTTTCTTAAAAA

TGAACAGTCTGCAAGCTGATGACACAGCCGTGTATTACTGTGCCAGAGATAAGGGATA

CTCCTATTACTATTCTATGGACTACTGGGGCCAAGGCACCACGGTCACCGTCTCCTCAG

GAGGCGGCGGCAGCGGCGGGGGAGGGTCCGGAGGGGGTGGTTCTGGTGGAGGAGG

ATCGGGAGGCGGTGGCAGCCAGGTCCAGCTGCAGCAGTCTGGGGCTGAACTGGCAA

GACCTGGGGCCTCAGTGAAGATGTCCTGCAAGGCTTCTGGCTACACCTTTACTAGGTA

CACGATGCACTGGGTAAAACAGAGGCCTGGACAGGGTCTGGAATGGATTGGATACATT

AATCCTAGCCGTGGTTATACTAATTACAATCAGAAGTTCAAGGACAAGGCCACATTGA

CTACAGACAAATCCTCCAGCACAGCCTACATGCAACTGAGCAGCCTGACATCTGAGG

ACTCTGCAGTCTATTACTGTGCAAGATATTATGATGATCATTACTGCCTTGACTACTGGG

GCCAAGGCACCACTCTCACAGTCTCCTCAGGTGGCGGTGGCTCCGGCGGTGGTGGGT

CGGGTGGCGGCGGATCTCAGATTGTGTTGACCCAGTCTCCAGCAATCATGTCTGCATC

TCCAGGGGAGAAGGTTACCATGACCTGCAGTGCCAGCTCAAGTGTAAGTTACATGAA

CTGGTACCAGCAGAAGTCAGGCACCTCCCCCAAAAGATGGATTTATGACACATCCAA

ACTGGCTTCTGGAGTCCCTGCTCACTTCAGGGGCAGTGGGTCTGGGACCTCTTACTCT CTCACAATCAGCGGCATGGAGGCTGAAGATGCTGCCACTTATTACTGCCAGCAGTGGA GTAGTAACCCATTCACGTTCGGCTCGGGGACCAAGCTGGAGATCAACCGTGCGGCCG CAACTACCACCTTCGAACATCCTCACATTCAAGACGCTGCTTCGCAACTTCCTGATGA TGAGAGTTTATTTTTTGGTGATACTGGGCTATCCAAAAATCCAATCGAGCTTGTAGAAG GTTGGTTCAGTAGTTGGAAAAGCTCTATTGCCTCTTTTTTCTTTATCATAGGGTTAATCA TTGGACTATTCTTGGTTCTCCGAGTTGGTATCCATCTTTGCATTAAATTAAAGCACACC AAGAAAAGACAGATTTATACAGACATAGAGATGAACCGACTTGGAAAG (SEQ ID NO: 34).

According to the embodiments of the present invention, the second nucleic acid molecule further comprises a nucleic acid sequence encoding a signal peptide. According to the specific embodiments of the present invention, the signal peptide expressed by the nucleic acid sequence encoding the signal peptide is located at the amino terminus of the fusion protein precursor protein and it is the membrane-localized telopeptide of the envelope protein. The signal peptide helps the envelope protein to locate to the endoplasmic reticulum, after the protein maturation, it is hydrolyzed and removed. Therefore, the fusion protein of the viral particle does not comprise this signal peptide.

According to the embodiments of the present invention, the nucleic acid sequence encoding a signal peptide has a nucleotide sequence shown in SEQ ID NO: 26.
ATGAAGTGCCTTTTGTACTTAGCCTTTTTATTCATTGGGGTGAATTGC (SEQ ID NO: 26).

According to the embodiments of the present invention, the ratio of the copy number of the first nucleic acid molecule and the second nucleic acid molecule is 1:1 ~ 4:1. It should be noted that the "ratio of the copy number of the first nucleic acid molecule and the second nucleic acid molecule" herein refers to the ratio of the number of the first nucleic acid molecule and the second nucleic acid molecule carried on the vector when the first viral vector and the second viral vector are the same vector, that is, when the first nucleic acid molecule and the second nucleic acid molecule are the same vector, so as to ensure that the ratio of the protein expression amount of the first nucleic acid molecule and the second nucleic acid molecule is approximately the same. The inventors found that when the ratio of the number of the first nucleic acid molecule and the second nucleic acid molecule carried on the vector is 1: 1 ~ 4:1, the virus titer and the infection efficiency of the virus are both higher.

According to the embodiments of the present invention, the ratio of the copy number of the first nucleic acid molecule and the second nucleic acid molecule is 2:1 ~ 4:1. According to the specific embodiments of the present invention, when the ratio of the number of the first nucleic acid molecule and the second nucleic acid molecule carried on the vector is 2:1 ~ 4:1, the virus titer and the infection efficiency of the virus are both further improved.

According to the embodiments of the present invention, the ratio of the copy number of the first nucleic acid molecule and the second nucleic acid molecule is 2:1. The inventors found that when the ratio of the number of the first nucleic acid molecule and the second nucleic acid molecule carried on the vector is 2:1, the virus titer and the infection efficiency of the virus will reach an optimal balance.

According to the embodiments of the present invention, the first viral vector and the second viral vector are the same vector.

According to the embodiments of the present invention, the first viral vector and the second viral vector are the same vector, the vector further comprises: an internal ribosome entry site sequence (IRES), wherein the internal ribosome entry site sequence is arranged between the first nucleic acid molecule and the second nucleic acid molecule. The expression of the two proteins before and after the internal ribosome entry site is usually proportional. The introduction of the internal ribosome entry site sequence allows the first nucleic acid molecule and the second nucleic acid molecule to be independently translated and expressed, and the resulting envelope protein and fusion protein are in a non-fusion form. The introduction of the internal ribosome entry site sequence effectively guarantees the biological effects of the envelope protein and the fusion protein, so that the specific binding adsorption and infectivity of the virus obtained by packaging is remarkable, and it can directly transduce T cells that have not been pre-activated, and the virus titer is high.

According to the embodiments of the present invention, the first viral vector and the second viral vector are the same vector, the vector further comprises: a third nucleic acid molecule, which is arranged between the first nucleic acid molecule and the second nucleic acid molecule, and the third nucleic acid molecule encodes a third linking peptide, and the third linking peptide can be cleaved. The introduction of the third nucleic acid molecule makes the expression of the envelope protein and the fusion protein in a non-fusion form, thereby ensuring the biological function of the envelope protein and the fusion protein, so that the specific binding attachment and infection ability of the virus obtained by packaging is remarkable, and it can directly transduce T cells that have not been pre-activated, and the virus titer is high.

According to the embodiments of the present invention, the first viral vector and the second viral vector are pMD2.G, pCMV, pMD2.G mutant or pCMV mutant. The types of the first viral vector and the second viral vector according to the embodiments of the present invention are not particularly limited, and a vector that can express VSV-G or a mutant of a vector that can express VSV-G or VSV-G mutant can be used.

According to the embodiments of the present invention, the viral vectors further comprises: a third viral vector and a fourth viral vector, the third viral vector carries gene of interest, and the fourth viral vector carries the viral structural protein genes and viral packaging enzyme gene and optional regulatory factor rev gene.

According to the embodiments of the present invention, the structural protein genes, the viral packaging enzyme gene and the regulatory factor rev gene are arranged on the same fourth viral vector or different fourth viral vectors. For example, the expression products of the lentiviral vector psPAX2 include structural protein gag, packaging enzyme pol (including reverse transcriptase, protease and integrase) and regulatory factor rev. wherein, the rev can increase the product titer to a certain extent, but it is not necessary for lentiviral packaging. Rev (pRSV-rev) and gag-pol (pMDLg-pRRE) can be divided into two plasmids for expression; or rev (pRSV-rev), gag (pCMV-gag), pol (pCMV-gag) can be divided into three plasmids for expression.

According to the embodiments of the present invention, the viral packaging enzyme comprises at least one of reverse transcriptase, protease, and integrase.

According to the embodiments of the present invention, the third viral vector is a transfer vector.

According to the embodiments of the present invention, the transfer vector comprises a lentiviral packaging signal.

According to the embodiments of the present invention, the lentiviral packaging signal comprises: Ψ.

According to the embodiments of the present invention, the transfer vector is a pLU vector.

According to the embodiments of the present invention, the fourth viral vector is psPAX2.

According to the embodiments of the present invention, the viral vectors are non-pathogenic virus.

According to the embodiments of the present invention, the third viral vector further carries gene of interest, and the gene of interest is a nucleic acid molecule encoding a chimeric antigen receptor.

In the second aspect of the present invention, provided herein is a method for obtaining lentivirus. According to the embodiments of the present invention, the method comprises: introducing the viral vectors provided herein into a first recipient cell; culturing the first recipient cell into which the viral vector is introduced to obtain a virus. The lentivirus obtained by the method has a high titer, and the abilities to target binding and infect cells are significantly improved, and it can directly transduce non-preactivated or pre-activated T cells.

According to the embodiments of the present invention, the method provided herein may further comprise at least one of the following additional technical features:

according to the embodiments of the present invention, the virus is lentivirus, the first viral vector and the second viral vector are different vectors, the mass ratio of the third viral vector, the fourth viral vector, the first viral vector and the second viral vector is 1:1:1:0.25 ~ 2:1:1:1. According to the ratio of viral vectors of the embodiments of the present invention, the lentivirus titer and lentivirus infection efficiency are both high.

According to the embodiments of the present invention, the mass ratio of the third viral vector, the fourth viral vector, the first viral vector and the second viral vector is 2:1:1:0.5.

According to the embodiments of the present invention, the mass ratio of the third viral vector, the fourth viral vector, the first viral vector and the second viral vector is 1:1:1:0.5.

According to the embodiments of the present invention, the mass ratio of the third viral vector, the fourth viral vector, the first viral vector and the second viral vector is 1:1:1:1.

The inventors found that when the mass ratio of the third viral vector, the fourth viral vector, the first viral vector and the second viral vector is 2:1:1:0.5, 1:1:1:0.5, 1 or 1:1:1;1, the titer and the infection efficiency of the lentivirus will reach an optimal balance.

According to the embodiments of the present invention, the first recipient cell is 293T.

In the third aspect of the present invention, provided herein is a lentivirus. According to the embodiments of the present invention, the lentivirus is obtained by packaging as described herein. The lentivirus according to the embodiments of the present invention has high titer and has the abilities to target binding and infect immune cells, and can directly transduce non-preactivated or pre-activated T cells.

In the fourth aspect of the present invention, provided herein is a lentivirus. According to the embodiments of the present invention, the lentivirus expresses an envelope protein and a fusion protein, the fusion protein comprises at least one single chain antibody and a C-terminal domain of the envelope protein. The single chain antibody is capable of binding to CD28 or CD3, and the C-terminal domain of the envelope protein comprises a transmembrane region and an intracellular region of the envelope protein, the C-terminal of the at least one single chain antibody is connected to the N-terminal of the C-terminal domain of the envelope protein. The lentivirus according to the embodiments of the present invention has high titer and has the abilities to target binding and infect immune cells, and can directly transduce non-preactivated or pre-activated T cells.

According to the embodiments of the present invention, the envelope protein is an envelope G glycoprotein or a mutant of envelope G glycoprotein of vesicular stomatitis virus.

In the fifth aspect of the present invention, provided herein is a lentivirus. According to the embodiments of the present invention, the lentivirus expresses envelope protein and fusion protein, the fusion protein comprises a first single chain antibody, a second single chain antibody and a C-terminal domain of the envelope protein. The first single chain antibody is capable of binding to CD28, the second single chain antibody is capable of binding to CD3, the C-terminal domain of the envelope protein comprises a transmembrane region and an intracellular region of the envelope protein. The C-terminal of the first single chain antibody is connected to the N-terminal of the second single chain antibody, and the C-terminal of the second single chain antibody is connected to the N-terminal of the C-terminal domain of the envelope protein; or , the C-terminal of the second single chain antibody is connected to the N-terminal of the first single chain antibody, and the C-terminal of the first single chain antibody is connected to the N-terminal of the C-terminal domain of the envelope protein. The lentivirus according to the embodiments of the present invention has high titer and has the abilities to target binding and infect immune cells, and can directly transduce non-preactivated or pre-activated T cells.

According to the embodiments of the present invention, the envelope protein is an envelope G glycoprotein or a mutant of envelope G glycoprotein of vesicular stomatitis virus.

In the sixth aspect of the present invention, provided herein is a method for introducing lentivirus into unactivated T lymphocytes. According to the embodiments of the present invention, the aforementioned lentiviral vectors are used to electroporate or transfect the unactivated T lymphocytes, or the aforementioned lentivirus are used to infect the unactivated T lymphocytes. As mentioned above, after the viral vectors are introduced into the recipient cells, a virus with high virus titer can be packaged, and the single chain antibody expressed by the virus, mediated by binding to CD28 or CD3, achieve the specific targeted binding of the virus to the immune cell, and then realize the infection of immune T cells to the virus, and according to the method of the embodiments of the present invention, lentivirus can be directly introduced into non-preactivated T cells in vivo or ex vivo.

In the seventh aspect, provided herein is a method for expressing gene of interest. According to the embodiments of the present invention, the method comprises: introducing the viral vectors or lentivirus integrated with the gene of interest into the second recipient cell; culturing the second recipient cell into which the viral vector or lentivirus is introduced to express the gene of interest. According to the embodiments of the present invention, the expression of the gene of interest in the recipient cells is effectively realized. For example, in the embodiments of the present invention, mCherry is a gene of interest that can be carried, in order to verify the feasibility of the targeting vector platform, the inventors used mcherry gene as a tag gene to express fluorescent protein, thereby characterizing the transduction positive rate of virus in the recipient cells.

According to the embodiments of the present invention, the method provided herein may further comprise at least one of the following additional technical features:

according to the embodiments of the present invention, the introduction into the second recipient cell is carried out by electrotransfection, transfection or infection. It should be noted that the "electrotransfection" or "transfection" refers to a method of introducing the viral vectors into a recipient cell, and the "infection" refers to a process in which the virus actively binds and fuses with the cell membrane to enter the cell. Wherein, "electrotransfection" refers to a method of introducing vectors for virus packaging into recipient cell by means of electrical stimulation, and "transfection" refers to a method of introducing vectors for virus packaging into recipient cell through chemical mediators, such as liposomes.

According to the embodiments of the present invention, the second receptor cell is an immune cell.

According to the embodiments of the present invention, the second receptor cell is a T cell. The expression of the gene of interest in T cells can achieve direct or indirect therapeutic effects. The method according to the embodiment of the present invention realizes the activation of immune cells, thereby strengthening the immune response.

In an eighth aspect of the present invention, the present invention provides a method of obtaining CAR-T cells. According to the embodiments of the present invention, the method comprises: introducing the aforementioned viral vectors or lentivirus integrated with chimeric antigen receptor encoding nucleic acid into T lymphocytes; culturing the T lymphocytes into which the viral vectors or lentivirus is introduced to express chimeric antigen receptor. As mentioned above, after the viral vector is introduced into the first recipient cells, a virus with high virus titer can be packaged, and the virus can express single chain antibodies. Therefore, the viral vector is directly introduced into T lymphocytes, or the packaged virus with high titer is introduced into T lymphocytes, the obtained single chain antibodies, when binding to CD28 or CD3, achieve the specific targeted binding of the virus to T lymphocytes, thereby achieving the infection of immune T cells. According to the method of the embodiments of the present invention, CAR-T cells can be obtained directly from non-preactivated or preactivated T lymphocytes ex vivo and in vivo, and the CAR-T cells can effectively inhibit the growth of tumor cells.

According to the embodiments of the present invention, the introduction into the T lymphocytes is carried out by electrotransfection, transfection or infection. It should be noted that the "electrotransfection" or "transfection" refers to a method of introducing the viral vectors into a recipient cell, and the "infection" refers to a process in which the virus actively binds and fuses with the cell membrane to enter the cell. Wherein, "electrotransfection" refers to a method of introducing vectors for virus packaging into recipient cell by means of electrical stimulation, and "transfection" refers to a method of introducing vectors for virus packaging into recipient cell through chemical mediators, such as liposomes.

According to the embodiments of the present invention, the "pMD2.G mutant" or "pMD2.G-Mut" both refers to a plasmid (VSV-G-K47Q\R354Q) expressing a lentiviral envelope protein containing K47Q\R354Q mutation sites.

In the ninth aspect of the present invention, provided herein is a CAR-T cell. According to the embodiments of the present invention, the CAR-T cell is prepared according to the method described in the eighth aspect of the present invention. The CAR-T cells prepared according to the embodiments of the present invention have low cost, high cell activity, high purity, and show good killing activity against tumor cells.

In the tenth aspect of the present invention, provided herein is a pharmaceutical composition. According to the embodiments of the present invention, the pharmaceutical composition comprises the viral vector described in the first aspect of the present invention, the lentivirus described in the fourth aspect of the present invention, or the CAR-T cell described in the eighth aspect of the present invention. As mentioned above, the CAR-T cell has the advantages of high cell activity, strong cell killing ability, and good immune activation effect. The delivery of the CAR-T cells into the body is conducive to the CAR-T cells to exert the killing ability on tumor cells.

In the eleventh aspect of the present invention, provided herein is use of the aforementioned viral vectors, lentiviruses, CAR-T cells and pharmaceutical compositions to activate immunity or treat or prevent diseases. The inventors found that the drugs comprising viral vectors, lentivirus or CAR-T cells have better effects on activating immunity, treating or preventing diseases.

In the twelfth aspect of the present invention, provided herein is use of the aforementioned viral vectors, lentiviruses, CAR-T cells and pharmaceutical compositions to treat or prevent tumors. The inventors found that the drugs comprising viral vectors, lentiviruses or CAR-T cells have superior efficacy on treating or preventing malignant tumors.

In the thirteenth aspect of the present invention, provided herein is a method for activating body immunity or treating or preventing diseases. According to the embodiments of the present invention, the method comprises administering to the subject a pharmaceutically acceptable amount of the aforementioned viral vectors, lentiviruses, CAR-T cells or pharmaceutical compositions. According to the embodiments of the present invention, the viral vectors, lentiviruses, CAR-T cells or pharmaceutical compositions can be used to activate immunity, treat or prevent diseases after administering to the subject in an effective amount.

According to the embodiments of the present invention, the disease is tumor.

## DESCRIPTION OF THE DRAWINGS

The above and/or additional aspects and advantages of the present invention will become apparent and readily understood from the description of the embodiments taken in conjunction with the following drawings, wherein:

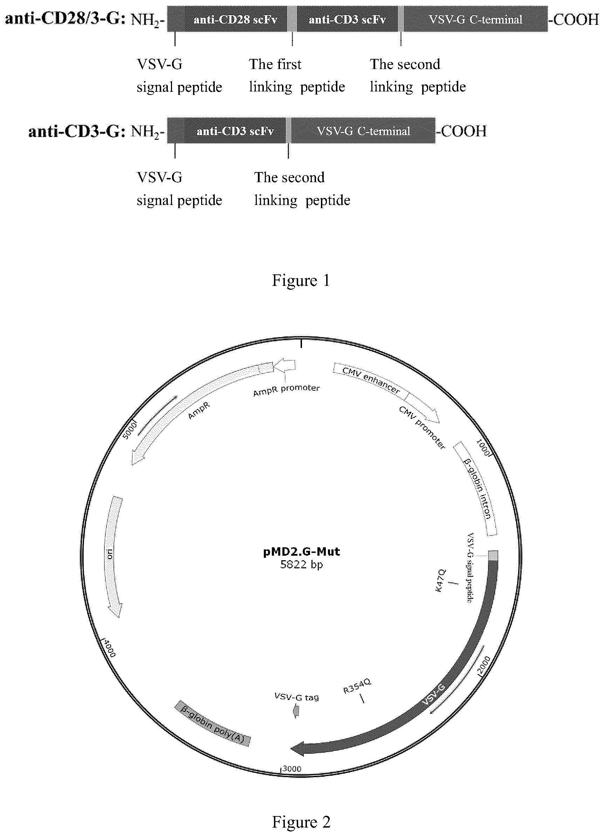

Figure 1 is a structural model diagram of envelope fusion proteins targeting CD28, CD3, and CD3 antigen (anti-CD28/3-G, anti-CD3-G) respectively according to the embodiments of the present invention, wherein, the anti-CD28scFv refers to the variable region sequence of the CD28 single chain antibody, Anti-CD3scFv refers to the variable region sequence of the CD3 single chain antibody, and VSV-G C-terminal refers to the C-terminal domain of the lentiviral envelope protein;

Figure 2 is a map of pMD2.G mutant (pMD2.G-Mut) according to the embodiments of the present invention;

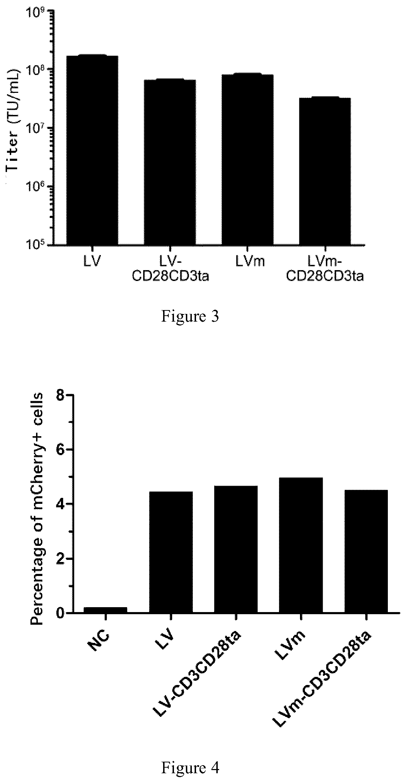

Figure 3 shows titer of the lentivirus with CD28 and CD3 targeting ability obtained by vector packaging under different conditions according to the embodiments of the present invention, wherein, the abscissa represents the type of lentivirus, the types and ratios of vectors contained in each lentivirus are shown in Table 1, the ordinate represents the lentivirus titer measured by the lentivirus infecting HEK-293T cells;

Figure 4 is a detection result diagram of the positive rate of HEK-293T cells infected with lentivirus targeting CD28 and CD3 obtained through different vector packaging according to the embodiments of the present invention, wherein, the abscissa represents the type of lentivirus, the types and ratios of vectors contained in each lentivirus are shown in Table 1, the ordinate represents the mCherry positive rate of HEK-293T cells infected by lentivirus;

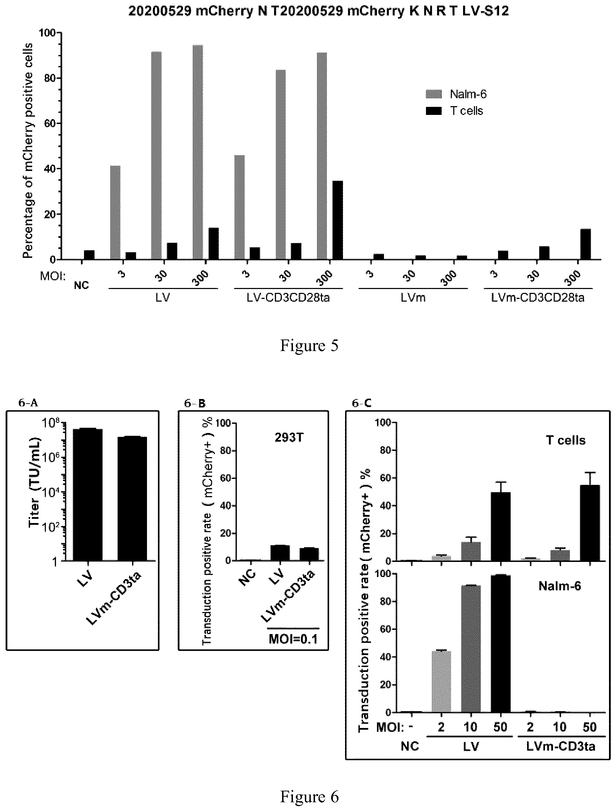

Figure 5 is an analysis diagram of the positive rate detection results of T cells and Nalm-6 cells transfected with different lentivirus according to the embodiments of the present invention, wherein, the abscissa represents the type of lentivirus, the types and ratios of vectors contained in each lentivirus are shown in Table 1, the ordinate represents the mCherry positive rate of HEK-293T cells infected by lentivirus;

Figure 6 is an analysis diagram of titer results of different lentivirus obtained by packaging according to the embodiments of the present invention, and an analysis diagram of the ability to infect 293T, T cells and Nalm-6 cells, wherein,

Figure 6-A shows the analysis of titer results of different lentivirus, the abscissa represents the type of lentivirus, the types and ratios of vectors contained in each lentivirus are shown in Table 2, the ordinate represents the titer of HEK-293T cells infected by lentivirus.

Figure 6-B shows the test results of the positive rate of HEK-293T cells transduced by different lentivirus, the abscissa represents the type of lentivirus, the types and ratios of vectors contained in each lentivirus are shown in Table 2, the ordinate represents the mCherry positive rate of HEK-293T cells infected by lentivirus;

Figure 6-C shows the detection results of the mCherry positive rate of T cells and Nalm-6 cells after the mixed system of T cells and Nalm-6 cells is infected by different lentivirus, the abscissa represents the type of lentivirus, the types and ratios of vectors contained in each lentivirus are shown in Table 2, the ordinate represents the mCherry positive rate of T cells and Nalm-6 cells infected by lentivirus;

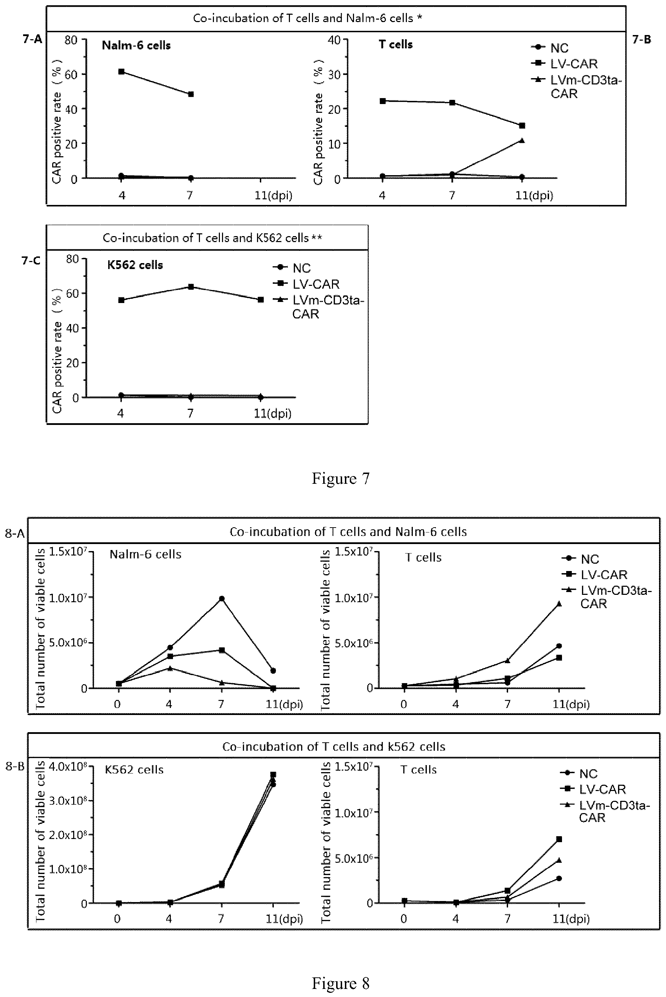

Figure 7 is a detection result diagram of the CAR positive rate of T cells, K562 cells and Nalm-6 cells after the mixed system of T cells and Nalm-6 or K562 cells is infected by different lentiviruses according to the embodiments of the present invention. The abscissa represents the type of lentivirus, the types and ratios of vectors contained in each lentivirus are shown in Table 3, the ordinate represents the CAR positive rate of T cells, K562 cells and Nalm-6 cells infected by lentivirus, wherein:

7-A and 7-B respectively represent the CAR positive rate of Nalm-6 cells and T cells after the mixed system of T cells and Nalm-6 cells is infected by different lentivirus,

7-C represents the CAR positive rate of K562 cells after the mixed system of T cells and K562 cells is infected by different lentivirus;

Figure 8 is a detection result diagram of the total number of viable cells of T cells, K562 cells and Nalm-6 cells after the mixed system of T cells and Nalm-6 or K562 cells is infected by different lentivirus according to the embodiments of the present invention. The abscissa represents the number of days after the mixed system of T cells and Nalm-6 or K562 cells is infected by lentivirus, the types and ratios of vectors contained in each lentivirus are shown in Table 3, the ordinate represents the total number of viable cells, wherein:

Figure 8-A shows the total number of viable cells of T cells and Nalm-6 cells after the mixed system of T cells and Nalm-6 cells is infected by lentivirus,

Figure 8-B shows the total number of viable cells of T cells and K562 cells after the mixed system of T cells and K562 cells is infected by lentivirus;

Figure 9 is an operational flow of the verification experiment of inoculating T cells and Nalm-6 cells into mice according to the embodiments of the present invention;

Figure 10 is a flow cytometric detection result diagram of mouse peripheral blood leukocytes after inoculation of T cells and Nalm-6 according to the embodiments of the present invention;

Figure 11 is a detection result analysis diagram of the positive rate of T cells transfected by different lentivirus according to the embodiments of the present invention;

Figure 12 is a titer result diagram of CAR-T prepared using lentiviral vectors according to the embodiments of the present invention;

Figure 13 is a diagram comparing the efficiency of transducing unactivated T cells with LV-CAR-2 and LV-S2-CAR vectors according to the embodiments of the present invention;

Figure 14 is a diagram comparing the efficiency of transducing activated T cells with LV-CAR-2 and LV-S2-CAR vectors according to the embodiments of the present invention;

Figure 15 is a diagram comparing the results of ex vivo CAR-T killing ability according to the embodiments of the present invention;

Figure 16 is a comparison of the in vivo efficacy of CAR-T products prepared based on different lentiviral vector processes;

Figure 17 is a comparison of the transduction efficiency of CD3+ Jurkat cells by lentiviral vectors packaged by CD3 targeting fusion proteins containing VSV-G C-terminal domains of different lengths

Figure 18 is a comparison of the titers of a lentiviral vector (LV-2) packaged by a fusion protein with scFv located at the N-terminal of complete VSV-G, and a lentiviral vector (LV-S2) packaged by VSV-G and a fusion protein with scFv at the C-terminal domain of VSV-G.

## EXAMPLES

Embodiments of the present invention are described in detail below, and examples of the embodiments are shown in drawings.

According to specific embodiments of the present invention, provided herein is a group of novel lentiviral vectors with targeted transfection ability, the group of vectors comprise a coding region carrying an envelope protein VSV-G or VSV-G mutant, and a coding region carrying a fusion protein obtained by linking the single chain antibody scFv and C-terminal domain of VSV-G through a connecting peptide.

The lentiviral vectors provided herein have the following characteristics: VSV-G mutant is a mutant that weakens the ability of VSV-G to attach to target cells, but retains the ability of cell membrane fusion; scFv can be one or more in series; the C-terminal domain of VSV-G at least includes the intracellular and transmembrane regions of VSV-G; a viral vector can contain one or more fusion proteins.

The advantages of this study: the fusion protein formed by scFv and the C-terminal domain of VSV-G has the same transmembrane and intracellular regions as VSV-G, so that the fusion protein maintains the interaction with the matrix protein, making it efficiently assembled on the envelope of the lentiviral particle without interfering with the budding of the virus particle, thereby not having a significant impact on virus titer. The envelope of the lentiviral particle still contains complete VSV-G or its mutant, which can maintain the stability of the virus particle. Through the binding of scFv to the corresponding antigen, the lentiviral particle can actively infect target cells which expresses corresponding antigens, and increase the infection efficiency of the target cells under the same infection multiplicity conditions. The scFv has a clear functional background and guaranteed safety, and any antigen can be screened to specifically bind scFv, making the virus packaged by the lentiviral vectors can be universally applied to the targeted infection of various types of cells. VSV-G is replaced with a mutant with weakened ability to adsorb target cells, so that the specific binding force between scFv and the corresponding antigen dominates the process of viral particle attachment, and further improves the targeting of recombinant lentivirus. The lentiviral vector constructed in the present invention can directly transduce unactivated blood cells, simplify the production process and reduce costs, and avoid the loss of drug efficacy caused by cell activation; such as CAR-T products prepared by directly transducing T cells, the ex vivo killing ability is strongest under the condition of low effective-target ratio.

According to specific embodiments of the present invention, provided herein is a method for constructing and using a new type of lentiviral vectors with the ability to gene of interest transfection, comprising the following steps (taking the lentiviral vector targeted for transfection of CD3⁺ cells as an example):

1. Designing a fusion protein (CD3scFv-VSV-G-CT (antiCD3-G for short)) formed by a single chain antibody targeting CD3 and the C-terminal domain of VSV-G. The structural pattern diagram is shown in Figure 1. Wherein, the VSV-G signal peptide is located at the N-terminal of the fusion protein precursor protein. The original VSV-G signal peptide is located at the amino terminus of the VSV-G precursor protein, and it is the membrane-localized telopeptide of the VSV-G protein, helping the VSV-G protein to locate to the endoplasmic reticulum, after the protein matures, it is hydrolyzed and removed, therefore, the mature VSV-G protein of the viral particle and the fusion protein do not contain this signal peptide.

The signal peptide has an amino acid sequence shown below:
MKCLLYLAFLFIGVNC (SEQ ID NO: 27).

The gene encoding the signal peptide has a nucleotide sequence shown below:
ATGAAGTGCCTTTTGTACTTAGCCTTTTTATTCATTGGGGTGAATTGC (SEQ ID NO: 26).

The amino acid sequence of anti-CD3 scFv is shown in SEQ ID NO:5.

DIKLQQSGAELARPGASVKMSCKTSGYTFTRYTMHWVKQRPGQGLEWIGY

INPSRGYTNYNQKFKDKATLTTDKSSSTAYMQLSSLTSEDSAVYYCARYYDDHYCLDYW

GQGTTLTVSSVEGGSGGSGGSGGSGGVDDIQLTQSPAIMSASPGEKVTMTCRASSSVSYM

NWYQQKSGTSPKRWIYDTSKVASGVPYRFSGSGSGTSYSLTISSMEAEDAATYYCQQWS

SNPLTFGAGTKLELK (SEQ ID NO: 5).

The amino acid sequence of the second linking peptide (Linker) is shown in SEQ ID NO: 12.

AAATTT (SEQ ID NO: 12).

VSV-G C-Terminal (VSV-G-CT) comprises amino acids 405-495 (91 in total) of the VSV-G protein, and the amino acid sequence is shown in SEQ ID NO: 13.

FEHPHIQDAASQLPDDESLFFGDTGLSKNPIELVEGWFSSWKSSIASFFFIIGLI

IGLFLVLRVGIHLCIKLKHTKKRQIYTDIEMNRLGK (SEQ ID NO:13).

2. Constructing CG fusion membrane protein expression plasmid

The DNA sequence was designed using the VSV-G signal peptide-scFv-Linker-(VSV-G-CT) pattern and was entrusted to a gene synthesis company (General Biosystems (Anhui) Co., Ltd.), and the pMD2.G plasmid (VSV-G protein expression plasmid) was used as a vector to construct the fusion protein expression plasmid pMD2.antiCD28/3-G, wherein the scFv comprises CD28 and/or CD3.

3. Constructing VSV-G weak attachment mutants (reference: Structural basis for the recognition of LDL-receptor family members by VSV glycoprotein. Nature Communications.2018)

The DNA sequence of K47Q and R354Q double point mutants was designed by a gene synthesis company. The VSV-G expression plasmid pMD2.G was used as a vector to construct a VSV-G-K47Q\R354Q expression plasmid pMD2.G-Mut. Studies have found that the mutation of K47Q and R354Q can only reduce the membrane attachment capacity of VSV-G, but not affect the membrane fusion ability of VSV-G.

4. Lentivirus packaging and harvest

① The lentiviral vector packaging plasmids were co-transfected into 293T cells.
② The virus-containing culture supernatant was collected after 48-72 h of transfection; filtered with a 0.45μm filter, PEG-concentrated directly or concentrated after purifying, then aliquoted and stored in an ultra-low temperature refrigerator.

5. Detection and analysis of targeted transfection ability of lentiviral vectors

① The gene of interest for transduction was mCherry: LUm-CD3ta-mCherry with its corresponding control lentiviral vector was measured the titer, transduced 293T cells, and transduced tumor cells and T cells, then the ability to specifically transduce T cells was evaluated.
② The gene of interest for transduction was anti-hCD19-CAR: After the previous step, the LUm-CD3ta vector was selected to load the anti-CD19-CAR gene of interest to obtain the lentiviral vector LUm-CD3ta-CAR. Together with the control vector, after titer determination, mixed T cells and tumor cells were transduced at MOI=1 (simulating the coexistence of T cells and tumor cells in the body). 4, 7 and 11 days after transduction, the cell concentration in each group, the ratio of T cells to tumor cells and CAR positive rate were detected respectively, then line chart of changes in CAR positive rate of each cell and growth curve of each cell were draw. Finally, the ability of LUm-CD3ta-CAR to target and transduce T cells to produce CAR-T and the ability of the produced CAR-T to kill tumor cells were evaluated.

6. Evaluating the ability of lentiviral vector LUm-CD3ta-CAR to target and transduce T cells to produce CAR-T and the ability of the produced CAR-T to kill tumor cells in mice.
Tumor cells and human T cells were mixed and injected into mice through the tail vein, and lentiviral vectors were injected into mice through the tail vein the next day. After 5 weeks, the content of tumor cells and T cells in the peripheral blood of the mice and the CAR positive rate of each cell were detected.
Embodiments of the present invention are described in detail below, and examples of the embodiments are shown in drawings. The embodiments described below with reference to the drawings are exemplary and are intended to explain the present invention and should not be construed as limiting the present invention.
It should be noted that the "plasmid" and "vector" described in the following embodiments have the same meaning and can be used interchangeably.

**Example 1: Lentiviral vector packaging and yield evaluation**

1.1 Packaging a series of lentiviruses that target T cells through CD28 and CD3 to transduce the gene of interest mCherry

1.1.1 Information of Lentivirus packaging

293T cells were co-transfected according to the plasmids and mass ratios shown in Table 1 (the quality of pLV-mCherry and psPAX2 plasmids between each group was maintained consistent) and lentivirus was packaged. Wherein, the pMD2. antiCD28/3-G plasmid expresses a fusion protein targeting CD3 and CD28 (antiCD28/3-G), and the pMD2.G-Mut plasmid expresses a mutant with reduced VSV-G attachment ability.

The gene encoding antiCD28/3-G has a nucleotide sequence shown below:
(SEQ ID NO:24).
The gene encoding antiCD28/3-G encodes a fusion protein with an amino acid sequence shown below:
(SEQ ID NO:16).

Table 1: Lentivirus packaging

| Lentivirus | Types and ratios of lentiviral vectors |
|---|---|
| LV(Lentivirus) | pLV-mCherry: psPAX2: pMD2.G=2:1:1 |
| LV-CD28CD3ta | pLV-mCherry: psPAX2: pMD2.G: pMD2.antiCD28/3-G =2:1:1:0.5 |
| LVm | pLV-mCherry: psPAX2: pMD2.G-Mut=2:1:1 |
| LVm-CD28CD3ta | pLV-mCherry: psPAX2: pMD2.G-Mut: pMD2.antiCD28/3-G =2:1:1:0.5 |

Wherein, pLV-mCherry is a transfer plasmid carrying mCherry sequence and the gene of interest is mCherry sequence, psPAX2 is a plasmid expressing lentiviral structural protein gag, non-structural protein pol and rev, pMD2.G is a plasmid expressing lentiviral membrane protein (VSV- G), pMD2.G-Mut is a plasmid expressing lentiviral membrane protein mutant (VSV-G-K47Q\R354Q), pMD2. antiCD28/3-G is a plasmid expressing a fusion protein (antiCD28/3-G) containing scFv targeting CD28 and CD3 and the C-terminal domain of VSV-G protein, wherein the C-terminal domain of VSV-G protein has an amino acid sequence shown in SEQ ID NO: 13, wherein the map of the pMD2.G mutant used in this example is shown in Figure 2.

1.1.2 Evaluation of lentiviral vector yield

1) Lentivirus packaging, harvesting, titer determination and T cell transfection

The lentiviral vectors obtained according to Table 1 were concentrated with PEG, aliquoted and frozen in an ultra-low temperature refrigerator (<-75 °C), and the titer was measured using 293T cells.
According to the experimental group in Table 1, 293T cells were transduced at MOI=0.05 as a positive control; viral vehicle was used as a negative control, and Nalm-6 (CD28$^-$CD3$^-$) and T cells (CD28$^+$CD3$^+$) were transduced, after transduction for 72 h, flow cytometry was used to detect the percentage of mCherry+ cells in the transduced cells.

2) Results and analysis

According to the results shown in Figure 3, there is no significant difference in the titers of the above four lentiviruses. The results in Figure 4 show that at MOI=0.05, the transduction positive rates of the four vectors in 293T cells were similar, indicating that the four vectors all have the ability to transduce cells. The results in Figure 5 show that in Nalm-6 and T cells, both LV and LU-CD28CD3ta have a high transduction positive rate and no transduction targeting, but the latter has a higher transduction ability for T cells than the former. LVm cannot transduce Nalm-6 cells and can only transduce a small amount of T cells. LVm-CD28CD3ta cannot transduce Nalm-6 cells, but it can transduce T cells, and its transduction ability is equivalent to that of LV, indicating that LVm-CD28CD3ta can target and transduce T cells.

**Example 2: Targeting efficiency evaluation of lentiviral vectors targeting CD3+ T cells**

2.1 Packaging a series of lentiviruses that transduce the gene of interest mCherry through CD3 targeted T cells.

According to the plasmids and ratios in Table 2 to co-transfect HEK 293T cells and package lentivirus.

Table 2: Lentivirus packaging

| Lentivirus | Types and ratios of lentiviral vectors |
|---|---|
| LU | pLU-mCherry: psPAX2: pMD2.G=2:1:1 |
| LUm-CD3ta | pLU-mCherry: psPAX2: pMD2.G-Mut: pMD2.antiCD3-G =2:1:1:0.5 |

Wherein, pLV-mCherry is a transfer plasmid carrying mCherry sequence and the gene of interest is mCherry sequence, psPAX2 is a plasmid expressing lentiviral structural protein gag, non-structural protein pol and rev, pMD2.G is a plasmid expressing lentiviral membrane protein (VSV-G), pMD2.G-Mut is a plasmid expressing lentiviral membrane protein mutant (VSV-G-K47Q\R354Q), pMD2. antiCD3-G is a plasmid (antiCD3-G) expressing a fusion protein containing scFv targeting CD3 and the C-terminal domain of VSV-G protein, wherein the C-terminal domain of the VSV-G protein has an amino acid sequence shown in SEQ ID NO: 13, wherein the map of the pMD2.G mutant used in this example is shown in Figure 2.

The gene encoding antiCD3-G has a nucleotide sequence shown below:
(SEQ ID NO: 23).

The gene encoding antiCD3-G has an amino acid sequence shown below:
(SEQ ID NO:15).

2.2 Targeting evaluation of lentiviral vectors targeting CD3+ T cells

1) Lentivirus packaging, harvesting, titer determination and target cell transfection

According to the experimental group in Table 2, 293T cells were co-transfected, the virus from each group were harvested 48-72 hours after transfection, then aliquoted and frozen in an ultra-low temperature refrigerator (<-75°C). Titer determination was performed using 293T cells.

The above two lentiviral vectors were used to transduce 293T cells at MOI=0.1 respectively, as a positive control; viral vehicles were used as a negative control, Nalm-6 (CD3⁻) and T cells (CD3⁺) were transduced at MOI=2, 10 and 50 respectively for 3-4 days, then the percentage of mCherry⁺ cells in the infected cells was detected by flow cytometry.

2) Results and analysis

The specific experimental results are shown in Figure 6. According to 6-A, there is no significant difference in the titers of the above two groups of lentiviruses. Figure 6-B shows that at MOI=0.1, the transduction positive rates of the above two vectors in 293T cells are similar, indicating that both vectors have the ability to transduce cells. Figure 6-C shows that LV can transduce both Nalm-6 and T cells, but LUm-CD3ta alone fails to transduce Nalm-6 cells, but can transduce T cells and the transduction ability is equivalent to that of LV, indicating that LUm-CD3ta can target transduce T cells.

**Example 3: Detection of transduction efficiency and tumor killing effect of lentiviral**

**vector targeting CD3+ T cells**

3.1 Packaging a series of lentiviruses that transduce the gene of interest anti-hCD19 scFv-CTM4O (CAR) through CD3 targeted T cells

According to the plasmids and ratios in Table 3 to co-transfect HEK 293T cells and package lentivirus.

Table 3: Lentivirus packaging

| Lentivirus | Types and ratios of lentiviral vectors |
|---|---|
| LU-CAR | pLV-CAR: pMDLg-pRRE: pRSV-rev: pMD2.G=1:1:1:1 |
| LVm-CD3ta-CAR | pLV-CAR: pMDLg-pRRE: pRSV-rev: pMD2.G-Mut: pMD2.antiCD3-G =1:1:1:1:0.5 |

Wherein, pLV-CAR is a transfer plasmid carrying CAR sequence, the gene of interest is anti-hCD19 scFv-CTM4O (CAR), pMDLg-pRRE is a plasmid expressing lentiviral structural protein gag and non-structural protein pol; pRSV-rev is a plasmid expressing the regulatory protein rev, pMD2.G is a plasmid expressing lentiviral envelope protein (VSV-G), pMD2.G-Mut is a plasmid expressing lentiviral membrane protein mutant (VSV-G-K47Q\R354Q), pMD2. antiCD3-G is a plasmid expressing a fusion protein containing scFv targeting CD3 and the C-terminal domain of VSV-G protein (antiCD3-G, the nucleotide sequence is shown in SEQ ID NO: 23, and the amino acid sequence is shown in SEQ ID NO: 15), wherein, the C-terminal domain of the VSV-G protein has an amino acid sequence shown in SEQ ID NO:13, wherein the map of the pMD2.G mutant used in this example is shown in Figure 1.

The gene encoding anti-hCD19 scFv- CTM4O (CAR) has a nucleotide sequence shown below (the bold part is the nucleotide sequence encoding Anti-hCD19 scFv):

**GACATCCAGATGACACAGACTACATCCTCCCTGTCTGCCTCTCTGGGAGAC
AGAGTCACCATCAGTTGCAGGGCAAGTCAGGACATTAGTAAATATTTAAATTGGT
ATCAGCAGAAACCAGATGGAACTGTTAAACTCCTGATCTACCATACATCAAGATT
ACACTCAGGAGTCCCATCAAGGTTCAGTGGCAGTGGGTCTGGAACAGATTATTC
TCTCACCATTAGCAACCTGGAGCAAGAAGATATTGCCACTTACTTTTGCCAACAG
GGTAATACGCTTCCGTACACGTTCGGAGGGGGGACCAAGCTGGAGATCACAGGT
GGCGGTGGCTCGGGCGGTGGTGGGTCGGGTGGCGGCGGATCTGAGGTGAAAC
TGCAGGAGTCAGGACCTGGCCTGGTGGCGCCCTCACAGAGCCTGTCCGTCACA
TGCACTGTCTCAGGGGTCTCATTACCCGACTATGGTGTAAGCTGGATTCGCCAG
CCTCCACGAAAGGGTCTGGAGTGGCTGGGAGTAATATGGGGTAGTGAAACCACA
TACTATAATTCAGCTCTCAAATCCAGACTGACCATCATCAAGGACAACTCCAAGA
GCCAAGTTTTCTTAAAAATGAACAGTCTGCAAACTGATGACACAGCCATTTACTA
CTGTGCCAAACATTATTACTACGGTGGTAGCTATGCTATGGACTACTGGGGCCAA
GGAACCTCAGTCACCGTCTCCTCA**GAATTCACCACGACGCCAGCGCCGCGACCAC
CAACACCGGCGCCCACCATCGCGTCGCAGCCCCTGTCCCTGCGCCCAGAGGCGTGCC
GGCCAGCGGCGGGGGGCGCAGTGCACACGAGGGGGCTGGACTTCGCCTGTGATATCT
ACATCTGGGCGCCCTTGGCCGGGACTTGTGGGGTCCTTCTCCTGTCACTGGTTATCAC
CCTTTACTGCAAGCGCGGCCGCAAGAAGCTGCTGTACATCTTCAAGCAGCCCTTCATG
CGCCCCGTGCAGACCACCCAGGAGGAGGACGGCTGTAGCTGCCGCTTCCCCGAGGA
GGAGGAGGGCGGCTGCGAGCTGCGGCGCGACCAGCGCCTGCCCCCCGACGCCCACA
AGCCCCCCGGCGGCGGCAGCTTCCGCACCCCCATCCAGGAGGAGCAGGCCGACGCC
CACAGCACCCTGGCCAAGATCCGCGTGAAATTTAGCCGCAGCGCCGACGCCCCCGCC
TACCAGCAGGGCCAGAACCAGCTGTACAACGAGCTGAACCTGGGCCGCCGCGAGGA
GTACGACGTGCTGGACAAGCGCCGGGGCCGCGACCCCGAGATGGGCGGCAAGCCCC
AGCGCCGCAAGAACCCCCAGGAGGGCCTGTACAACGAGCTGCAGAAGGACAAGATG
GCCGAAGCCTACAGCGAGATCGGCATGAAGGGCGAGCGCCGCCGGGGCAAGGGCCA
CGACGGCCTGTACCAGGGCCTGAGCACCGCCACCAAGGACACCTACGACGCCCTGCA
C (SEQ ID NO: 28).

Anti-hCD19 scFv- CTM4O (CAR) has an amino acid sequence shown below (the bold part is the sequence of Anti-hCD19 scFv):

**DIQMTQTTSSLSASLGDRVTISCRASQDISKYLNWYQQKPDGTVKLLIYHTSRL**

**HSGVPSRFSGSGSGTDYSLTISNLEQEDIATYFCQQGNTLPYTFGGGTKLEITGGGGS**

**GGGGSGGGGSEVKLQESGPGLVAPSQSLSVTCTVSGVSLPDYGVSWIRQPPRKGLE**

**WLGVIWGSETTYYNSALKSRLTIIKDNSKSQVFLKMNSLQTDDTAIYYCAKHYYYG**

**GSYAMDYWGQGTSVTVSS**EFTTTPAPRPPTPAPTIASQPLSLRPEACRPAAGGAVHTRGL

DFACDIYIWAPLAGTCGVLLLSLVITLYCKRGRKKLLYIFKQPFMRPVQTTQEEDGCSCRF

PEEEEGGCELRRDQRLPPDAHKPPGGGSFRTPIQEEQADAHSTLAKIRVKFSRSADAPAYQ

QGQNQLYNELNLGRREEYDVLDKRRGRDPEMGGKPQRRKNPQEGLYNELQKDKMAEA

YSEIGMKGERRRGKGHDGLYQGLSTATKDTYDALH (SEQ ID NO: 29).

### 3.2 Lentiviral vector transduction

T cells were co-cultured with tumor cells Nalm-6 or K562 (number of cells, T cells: tumor cells = 1:2), and were transduced with lentiviral vectors (MOI = 1), an equal volume of vehicle was used as a negative control. On days 4, 7 and 11 after transduction, the number of cells in each group was measured to draw a growth curve, and the CAR positive rate of each cell was measured to evaluate the transduction function of the vector targeted T cells. The tumor cells Nalm-6 are CD19+ cells that can be effectively killed by anti-CD19 CAR-T and stimulate CAR-T cell proliferation; K562 are CD19- control cells that cannot be killed by anti-CD19 CAR-T and cannot stimulate CAR-T cell proliferation. The number of cells used to draw the cell growth curve is calculated as follows:

Cell number = cell density $\times$ volume $\times$ cell proportion corresponding to flow cytometry.

### 3.3 Result analysis

Figure 7 presents the aggregated data on the positivity rates across different cell populations. While LV-CAR showcases robust transduction efficacy towards T cells and the tumor cell lines Nalm-6 or K562, it falls short in terms of targeted transduction specificity. On the other hand, LUm-CD3ta excels in precisely targeting T cells for transduction, the CAR-T positivity rate of around 10% by the eleventh day. Prior to day 7, CAR-T cells continue to eliminate tumor cells. Although these CAR-T cells are capable of specific proliferation, they also undergo significant cell death concurrently. Consequently, no CAR-positive signals are detectable on days 4 and 7. Post day 7, once all tumor cells have been eradicated, the CAR-T cells persist in proliferating specifically. This leads to an increase in the positivity rate, which reaches approximately 10% by day 11.

The growth curve of each cell is shown in Figure 8. Under the co-incubation conditions of T cells and K562 (as shown in Figure 8-B), lentiviral vector transduction will not affect the growth rate of K562 and T cells. Under the co-incubation conditions of T cells and Nalm-6 (as shown in Figure 8-A), the growth rate of Nalm-6 in the LUm-CD3ta-CAR transduction group was the slowest before day 4, and the number of Nalm-6 cells began to decrease sharply after day 4, the strongest tumor killing effect was first demonstrated; the number of Nalm-6 cells in the LV transduction group was still increasing before day 7, but the growth rate was higher than the LUm-CD3ta group and lower than the NC group, indicating that the growth of tumor cells was inhibited, but the effect is not as good as LUm-CD3ta; the number of Nalm-6 cells in the LV transduction group began to decrease sharply to 0 after day 7, but the number of Nalm-6 cells in the NC group also began to decrease sharply during this period, indicating that most of the decrease in tumor cell numbers during this period is caused by the effect of non-CAR-T-dependent tumor killing.

In summary, under co-incubation conditions of T cells and tumor cells, which simulates the conditions in which T cells and tumor cells coexist in animals at a certain extent, LUm-CD3ta can achieve targeted transduction of T cells and generate CAR-T cells to kill tumor cells efficiently and specifically.

**Example 4: Evaluation of tumor killing function of LVm-CD3ta-CAR in hematologic malignancy mouse model**

4.1 Functional evaluation of lentiviral vector LUm-CD3ta-CAR in NCG mice Nalm-6 hematoma model

Functional evaluation was performed based on the lentiviral vector LUm-CD3ta-CAR obtained in Example 3. The specific experimental steps are as follows:

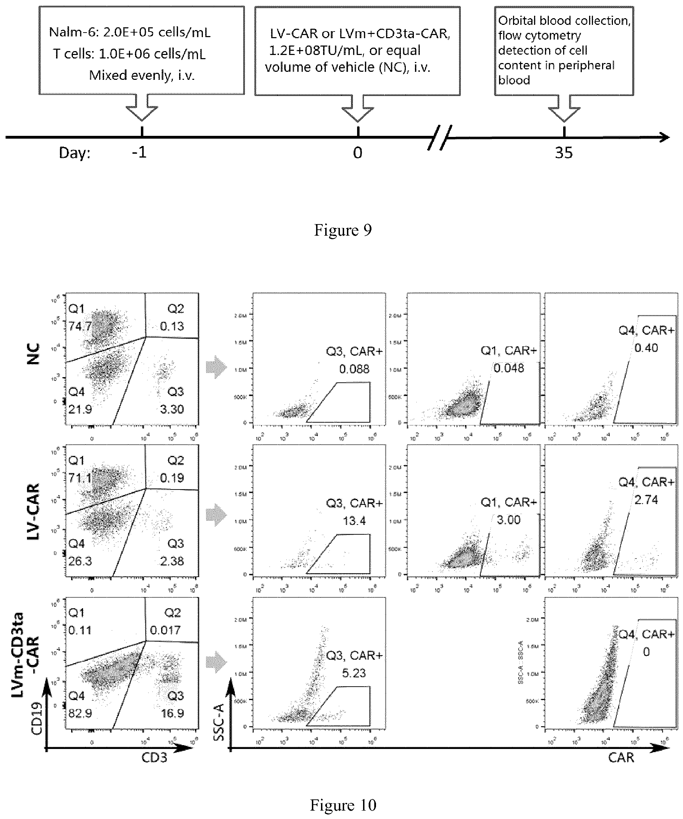

1) After NCG mice passed the quarantine, vaccination plan was arranged, the detailed experimental process is shown in Figure 9. The specific implementation method of T cells and tumor cells is as follows:

The Nalm-6 cells were subjected to cell counting and viability testing. When the viability was above 95%, the cells could be used for inoculation; the culture medium was removed by centrifugation, and PBS was added to adjust the cell density to 2.0E+06 cells/mL. After resuscitating the T cells frozen in liquid nitrogen, cell counting and viability testing were performed. When the viability was above 80%, the cells could be used for injection; PBS was added to adjust the cell density to 1.0E+07 cells/mL; the adjusted cell density of Nalm-6 and T cells suspensions were mixed evenly at a volume of 1:1, and 200 μL/mouse was injected through the tail vein of mice. The injection time point was recorded as day-1.

2) Lentiviral vector administration:

The mice that were inoculated with cells the day before were randomly divided into 3 groups. LV-CAR and LUm-CD3ta-CAR were diluted to 6.0E+07 TU/mL with lentiviral vector vehicle respectively; the mice were administered through the tail vein, and the NC group was infused with vehicle at 200 μL/mouse, the LV-CAR group was infused with LV-CAR dilution at 200 μL/mouse, and the LUm-CD3ta-CAR group was infused with LUm-CD3ta-CAR dilution at 200 μL/mouse; the infusion time point was recorded as day0.

3) Detection of tumor cells and CAR-T cells in the peripheral blood of mice

On Day 35, blood was collected from the orbit of mice in each group to obtain erythroblast lysed red blood cells → cell death dye\antibody incubation → flow cytometry was used to detect the proportion of each cell.

4.2 Result analysis

The experimental results are shown in Figure 10. The Q1 gate contains hCD19⁺hCD3⁻ cells, which are Nalm-6 tumor cells; the Q2 gate contains cells of no concern; the Q3 gate contains hCD19⁻hCD3⁺ cells, which are inoculated human T cells; the Q4 gate contains hCD19⁻hCD3⁻ cells, which are other white blood cells in the mouse blood.

In the NC group, it was found that 74.7% of the leukocytes in the peripheral blood of mice were Nalm-6 cells and 3.3% were T cells, indicating that Nalm-6 grew rapidly and had good tumorigenicity.

In the LV-CAR group, 71.1% of the leukocytes in the peripheral blood of mice were Nalm-6 cells and 2.38% were T cells, which has no significant difference with the NC group, and CAR positive cells were detected in all cell groups, indicating that LU-CAR transduces cells in mice in a non-specific manner, and the generated CAR-T cells cannot effectively kill tumor cells.

In the LUm-CD3ta-CAR group, no Nalm-6 cells were detected in the peripheral blood leukocytes of mice, and 16.9% were T cells. CAR positive cells were detected only in T cells, indicating that LUm-CD3ta-CAR can target transduction of T cells in mice, and the generated CAR-T cells can efficiently kill Nalm-6 tumor cells and stimulate the specific proliferation of CAR-T cells in the process.

In summary, in the Nalm-6 hematologic malignancy NCG mouse model, intravenous administration of LUm-CD3ta-CAR can achieve targeted transduction of T cells and generate CAR-T cells with normal functions, reflecting the anti-tumor efficacy.

**Example 5: Construction of a series of mCherry-loaded lentiviral vectors to transduce T cells**

5.1 Information of lentivirus packaging

The plasmids and mass ratios shown in Table 4 of the present invention were used to co-transfect 293T cells and package lentivirus.

Table 4 Lentiviral vector packaging plasmids and mass ratios

| Lentiviral vector | Plasmids and mass ratios |
|---|---|
| LV-mCherry | pLU-mCherry: pMDLg-pRRE; pRSV-rev: pMD2.G=1:1:1:1 |
| LU-S2-mCherry | pLU-mCherry: pMDLg-pRRE: pRSV-rev: pMD2.G: pMD2.S2=1:1:1:1:0.5 |

(continued)

| Lentiviral vector | Plasmids and mass ratios |
|---|---|
| LV-S12-mCherry | pLV -mCherry: pMDLg-pRRE: pRSV-rev: pMD2.G: pMD2.S12=1:1:1:1:0.5 |
| LV-S3-mCherry | pLV-mCherry: pMDLg-pRRE: pRSV-rev: pMD2.G: pMD2.S3=1:1:1:1:0.5 |
| LV-S4-mCherry | pLV-mCherry: pMDLg-pRRE: pRSV-rev: pMD2.G: pMD2.S4=1:1:1:1:0.5 |
| LV-S34-mCherry | pLV -mCherry: pMDLg-pRRE: pRSV-rev: pMD2.G: pMD2.S34=1:1:1:1:0.5 |

Among them, pLV-mCherry is a transfer plasmid carrying the mCherry sequence; pMDLg-pRRE is a packaging plasmid expressing the lentiviral structural protein *gag* and non-structural protein pol; pRSV-rev is a regulatory plasmid expressing the regulatory protein rev; pMD2.G is an envelope plasmid expressing envelope protein VSV-G; pMD2.S2/ pMD2.S12/ pMD2.S3/ pMD2.S4/ pMD2.S34 is a plasmid expressing the fusion protein S2/S12/S3/S4/S34 containing scFv targeting CD3, CD28 or CD3 and CD28 and the C-terminal domain of VSV-G protein respectively, wherein the C-terminal domain of VSV-G protein has an amino acid sequence shown in SEQ ID NO: 13, and the fusion protein S2 comprises the amino acid sequence shown in SEQ ID NO: 15, the fusion protein S12 comprises the amino acid sequence shown in below, the fusion protein S3 comprises the amino acid sequence shown in SEQ ID NO:18, the fusion protein S4 comprises the amino acid sequence shown in SEQ ID NO:19, the fusion protein S34 comprises the amino acid sequence shown in SEQ ID NO: 20.

5.2 Harvesting and storage of lentiviral vectors

48-72 h after transfection, the culture supernatant containing virus was collected, then filtered with a 0.45 $\mu$m filter, concentrated with PEG, aliquoted and stored in an ultra-low temperature refrigerator (<-75°C).

5.3 Evaluation of T cells transfected with lentiviral vectors

The above lentiviral vectors were used to transduce T cells respectively. 7 days after transduction, the mCherry expression positive rate in each group was detected by flow cytometry, that is, the transduction positive rate.

5.4 Results and analysis

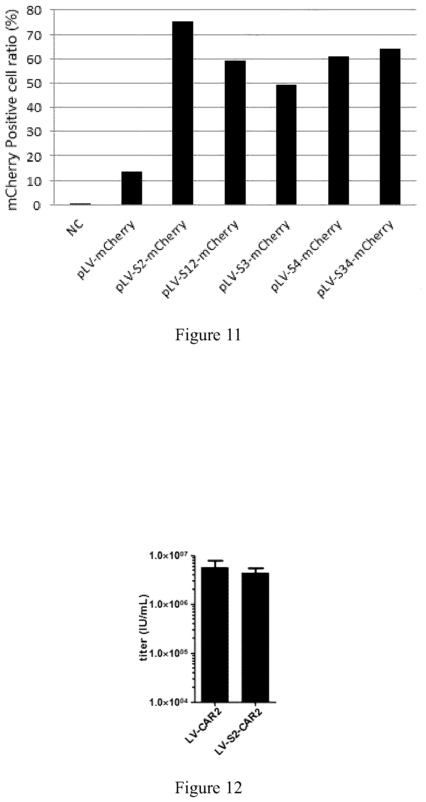

The results are shown in Figure 11, wherein NC is the negative control group using a vehicle without lentiviral vector during transduction.
The results in Figure 11 show that a series of fusion proteins containing scFv that specifically bind to T cell surface antigens (CD3\CD28) are packaged on the surface of lentiviral vectors, as membrane proteins, which is designed to improve the transduction efficiency of T cells by lentiviral vectors loaded with mCherry genes. Wherein, the transduction positive rate of LV-S2-mCherry, LV-S12-mCherry, LV-S3-mCherry, LV-S4-mCherry and LV-S34-mCherry increased significantly, which is more than 3 times that of LV-mCherry.

**Example 6: Construction of lentiviral vector loaded with anti-CD19-CAR (referred to as CAR) for transduction of T cells**

6.1 Based on the outcomes from Example 5, the lentiviral vectors that demonstrated the most substantial increase in transduction positive rate were selected for further use in loading the CAR gene to evaluate transduction efficiency. Utilizing the plasmids and their corresponding mass ratios detailed in Table 5, 293T cells were co-transfected to package the specified lentiviral vectors.

Table 5 Lentiviral vector packaging plasmids and mass ratios

| Lentiviral vector | Plasmids and mass ratios |
|---|---|
| LV-CAR2 | pLV-CAR2: pMDLg-pRRE: pRSV-rev: pMD2.G=1:1:1:1 |
| LV-S2- CAR2 | pLV-CAR2: pMDLg-pRRE: pRSV-rev: pMD2.G: pMD2.S2=1:1:1:1:0.5 |

Wherein, pLV-CAR2 is a transfer plasmid carrying CAR sequence and carries the gene of interest anti-hCD19 scFv- CAR;

the gene encoding anti-hCD19 scFv- CAR2 has a nucleotide sequence shown in SEQ ID NO:30:

GACATCCAGATGACACAGACTACATCCTCCCTGTCTGCCTCTCTGGGAGACAGAG

TCACCATCAGTTGCAGGGCAAGTCAGGACATTAGTAAATATTTAAATTGGTATCAGCAG

AAACCAGATGGAACTGTTAAACTCCTGATCTACCATACATCAAGATTACACTCAGGAG

TCCCATCAAGGTTCAGTGGCAGTGGGTCTGGAACAGATTATTCTCTCACCATTAGCAA

CCTGGAGCAAGAAGATATTGCCACTTACTTTTGCCAACAGGGTAATACGCTTCCGTAC

ACGTTCGGAGGGGGGACCAAGCTGGAGATCACAGGTGGCGGTGGCTCGGGCGGTGG

TGGGTCGGGTGGCGGCGGATCTGAGGTGAAACTGCAGGAGTCAGGACCTGGCCTGG

TGGCGCCCTCACAGAGCCTGTCCGTCACATGCACTGTCTCAGGGGTCTCATTACCCGA

CTATGGTGTAAGCTGGATTCGCCAGCCTCCACGAAAGGGTCTGGAGTGGCTGGGAGT

AATATGGGGTAGTGAAACCACATACTATAATTCAGCTCTCAAATCCAGACTGACCATCA

TCAAGGACAACTCCAAGAGCCAAGTTTTCTTAAAAATGAACAGTCTGCAAACTGATG

ACACAGCCATTTACTACTGTGCCAAACATTATTACTACGGTGGTAGCTATGCTATGGAC

TACTGGGGCCAAGGAACCTCAGTCACCGTCTCCTCAGAATTCACCACGACGCCAGCG

CCGCGACCACCAACACCGGCGCCCACCATCGCGTCGCAGCCCCTGTCCCTGCGCCCA

GAGGCGTGCCGGCCAGCGGCGGGGGGCGCAGTGCACACGAGGGGGCTGGACTTCGC

CTGTGATATCTACATCTGGGCGCCCTTGGCCGGGACTTGTGGGGTCCTTCTCCTGTCAC

TGGTTATCACCCTTTACTGCTGTTGGCTTACAAAAAAGAAGTATTCATCCAGTGTGCAC

GACCCTAACGGTGAATACATGTTCATGAGAGCAGTGAACACAGCCAAAAAATCTAGA

CTCACAGATGTGACCCTAAAGCGCGGCCGCAAGAAGCTGCTGTACATCTTCAAGCAG

CCCTTCATGCGCCCCGTGCAGACCACCCAGGAGGAGGACGGCTGTAGCTGCCGCTTC

CCCGAGGAGGAGGAGGGCGGCTGCGAGCTGCGCGTGAAATTTAGCCGCAGCGCCGA

CGCCCCCGCCTACCAGCAGGGCCAGAACCAGCTGTACAACGAGCTGAACCTGGGCCG

CCGCGAGGAGTACGACGTGCTGGACAAGCGCCGGGGCCGCGACCCCGAGATGGGCG

GCAAGCCCCAGCGCCGCAAGAACCCCCAGGAGGGCCTGTACAACGAGCTGCAGAAG

GACAAGATGGCCGAAGCCTACAGCGAGATCGGCATGAAGGGCGAGCGCCGCCGGGG

CAAGGGCCACGACGGCCTGTACCAGGGCCTGAGCACCGCCACCAAGGACACCTACG

ACGCCCTGCACATGCAGGCCCTGCCCCCCGC (SEQ ID NO: 30).

Anti-hCD 19 scFv-CAR2 has an amino acid sequence shown in SEQ ID NO:31:

DIQMTQTTSSLSASLGDRVTISCRASQDISKYLNWYQQKPDGTVKLLIYHTSRLHSG

VPSRFSGSGSGTDYSLTISNLEQEDIATYFCQQGNTLPYTFGGGTKLEITGGGGSGGGGGSG

GGGSEVKLQESGPGLVAPSQSLSVTCTVSGVSLPDYGVSWIRQPPRKGLEWLGVIWGSET

TYYNSALKSRLTIIKDNSKSQVFLKMNSLQTDDTAIYYCAKHYYYGGSYAMDYWGQGT

SVTVSSEFTTTPAPRPPTPAPTIASQPLSLRPEACRPAAGGAVHTRGLDFACDIYIWAPLAG

TCGVLLLSLVITLYCCWLTKKKYSSSVHDPNGEYMFMRAVNTAKKSRLTDVTLKRGRKK

LLYIFKQPFMRPVQTTQEEDGCSCRFPEEEEGGCELRVKFSRSADAPAYQQGQNQLYNEL

NLGRREEYDVLDKRRGRDPEMGGKPQRRKNPQEGLYNELQKDKMAEAYSEIGMKGER

RRGKGHDGLYQGLSTATKDTYDALHMQALPPR (SEQ ID NO: 31).

6.2 Harvesting, purification and storage of lentiviral vectors

48-72 h after transfection, the culture supernatant containing virus was collected, then filtered with a 0.45 $\mu$m filter, some samples were used as stock solution samples for titer determination; the remaining samples were purified and concentrated, then aliquoted and stored in an ultra-low temperature refrigerator (<-75°C).

6.3 Titer determination of lentiviral vectors

a. 293T cells were inoculated into a 24-well plate at an appropriate confluence and cultured overnight to allow cells to adhere;
b. The sample was taken out and the virus sample was diluted through a 2-fold gradient dilution method, and 8 $\mu$g/mL polybrene was added to the diluted virus solution;
c. The culture medium was removed from the 293T culture plate, and the cells in any two wells were counted after trypsin digestion, and the average number of cells in each well (A) was calculated; the original culture medium in the remaining wells was discarded, then 0.4 mL of virus diluent was added, the mixture was centrifuged for 40 min at 1000$\times$ g at room temperature, then incubated at 37°C, 5% $CO_2$ for 16 h, and then replaced the culture medium with fresh DMEM containing 8% FBS;
d. After 72 h of culturing, the positive rate of CAR expression was detected by flow cytometry (B);
e. Calculation of titer: the values of wells with a positive rate of CAR expression between 2% and 20% were selected for calculation: titer (PFU/mL) = B*A/(0.4/corresponding dilution factor).

6.4 Evaluation of positive rates post transduction of T cells transfected with lentiviral vectors

The above lentiviral vectors were used to transduce unactivated T cells or activated T cells at MOI=2 respectively. 2, 4 and 7 days after transduction, the positive rate of CAR expression of cells in each group was detected by flow cytometry, that is, the transduction positive rate.

6.5 Comparison of killing abilities of CAR-T cells transduced by different lentiviral vectors

a. The cells were inoculated as shown in Table 6, and cultured statically at 37°C and 5% $CO_2$;

Table 6 Schematic table of Nalm6 cells inoculating

| | T(NegCtrl)+Nalm6 (control group) | | | CAR T (No.1)+Nalm6 (experimental group) | | | CAR T (No.2) +Nalm6 (experimental group) | | |
| --- | --- | --- | --- | --- | --- | --- | --- | --- | --- |
| Target+lysis | + | + | + | + | + | + | + | + | + |

(continued)

|  | T(NegCtrl)+Nalm6 (control group) | | | CAR T (No.1)+Nalm6 (experimental group) | | | CAR T (No.2) +Nalm6 (experimental group) | | |
|---|---|---|---|---|---|---|---|---|---|
| Target+NC | + | + | + | + | + | + | + | + | + |
| OnlyT, E/T=0.1 | + | + | + | + | + | + | + | + | + |
| E/T=0.1 | + | + | + | + | + | + | + | + | + |
| OnlyT, E/T=0.2 | + | + | + | + | + | + | + | + | + |
| E/T=0.2 | + | + | + | + | + | + | + | + | + |
| Note: The K562 tumor cells in Table 6 are plated as above; target: target cells; E, effector cells, CAR-T cells; T, target cells, Nalm6 or K562 cells; "target+lysis" and "target+NC" are only plated Target cells; T (NegCtrl): T cells that has not been transduced. | | | | | | | | | |

b. After incubating for 18-20 h, 20 μl of lysis buffer was added to each well of the [target + lysis] group, the mixture was mixed thoroughly, and lysed in a cell incubator at 37°C for 30 min;

c. All culture medium was gently transferred to a new round-bottom 96-well plate and centrifuged at 400×g for 5 min at room temperature. 100 μL of supernatant from each well was taken to a flat-bottom 96-well plate for LDH cell killing detection, Cytotoxicity LDH assay kit (DOJINDO MOLECULAR, CK12) was used;

d. 100 μL of working solution was added to each well and mixed thoroughly, and incubated at 37°C for 30-60 min; then 50 μL of stop solution was added to each well and mixed thoroughly, and the absorbance value at 490 nm was measured;

e. Data processing:

$$\text{Positive control (PS)} = (\text{target} + \text{lysis}) - (\text{target} + \text{NC})$$

$$\text{Experimental group kill (EFF)} = (\text{E/T=n}) - (\text{onlyT, E/T=n})$$

$$\text{Cytotoxicity} = 100\% * (\text{EFF}) / (\text{PS})$$

6.6 Results and analysis

1) CAR-T titer detection

The comparison results of the original solution titers are shown in Figure 12. According to the results in Figure 12, it can be seen that there is no significant difference between the production titers of LV-CAR2 and LV-S2-CAR2 vectors.

2) Transduction efficiency of CAR-encoding lentiviral vectors into unactivated T cells

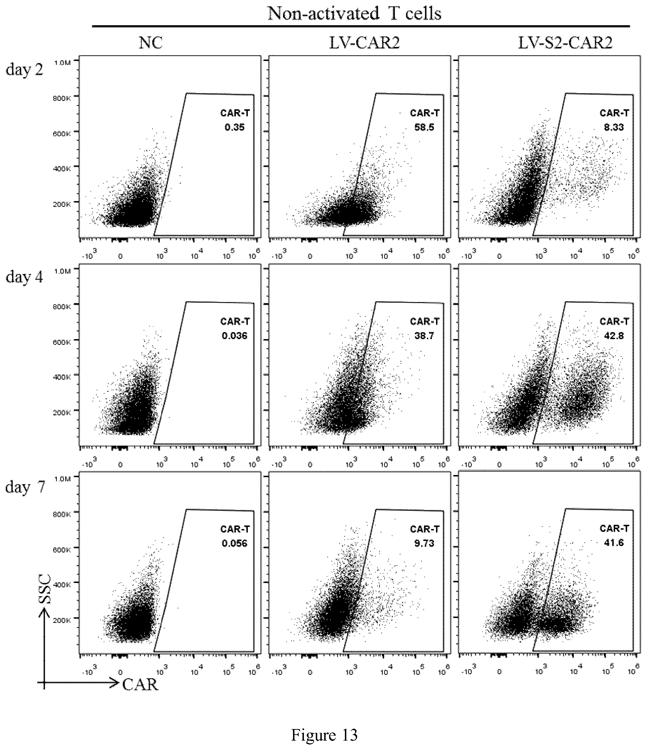

LV-CAR2 and LV-S2-CAR2 lentiviruses were used to transduce unactivated T cells at MOI=2 respectively, wherein NC is the negative control group (Negative Control) that adds vehicle without lentiviral vector during transduction. At 2 days (day 2), 4 days (day 4), and 7 days (day 7) after transduction, the positivity rate of CAR expression of each group was detected by flow cytometry. The comparison of the positivity rate of CAR expression of each group are shown in Figure 13;

Figure 13 illustrates a comparative analysis of transduction efficiencies between unactivated T cells using the LV-CAR2 and LV-S2-CAR2 vectors. Initially, the LV-CAR2 vector demonstrates a substantial presence of CAR-positive cells; however, the majority exhibit weak CAR signals. As cultivation time progresses, the proportion of CAR-positive cells markedly declines (Day 2: 58.5%, Day 4: 38.7%, Day 7: 9.73%), suggesting that the weak CAR signals do not originate from the stable integration of genes into the host cell genome for sustained expression. Hence, employing the Day 7 positivity rate provides a more precise evaluation of LU-CAR2's transduction efficacy on unactivated T cells. Upon transduction with the LV-S2-CAR2 vector, the percentage of CAR-positive cells escalates and stabilizes post-Day 4 (Day 2: 8.33%, Day 4: 42.8%, Day 7: 41.6%). This pattern could be attributed to the delayed kinetics of reverse transcription and integration processes within unactivated T cells, coupled with subdued protein expression. Consequently, the CAR expression levels on Day 2 might be insufficient for effective detection. The CAR positive cell rate on day 7, LV-S2-CAR2 was 4.28 times higher than that of LV-CAR2.

The aforementioned outcomes unequivocally demonstrate that conventional lentiviral vectors, such as LV, exhibit remarkably low efficacy in delivering CAR genes into unactivated T cells, while the LU-S2 lentiviral vector manifests a profound capability to transduce unactivated T cells with high efficiency, and exhibit stable and sustained expression of the CAR molecule.

3) Assessment of CAR Transduction Efficiency in Activated T Cells

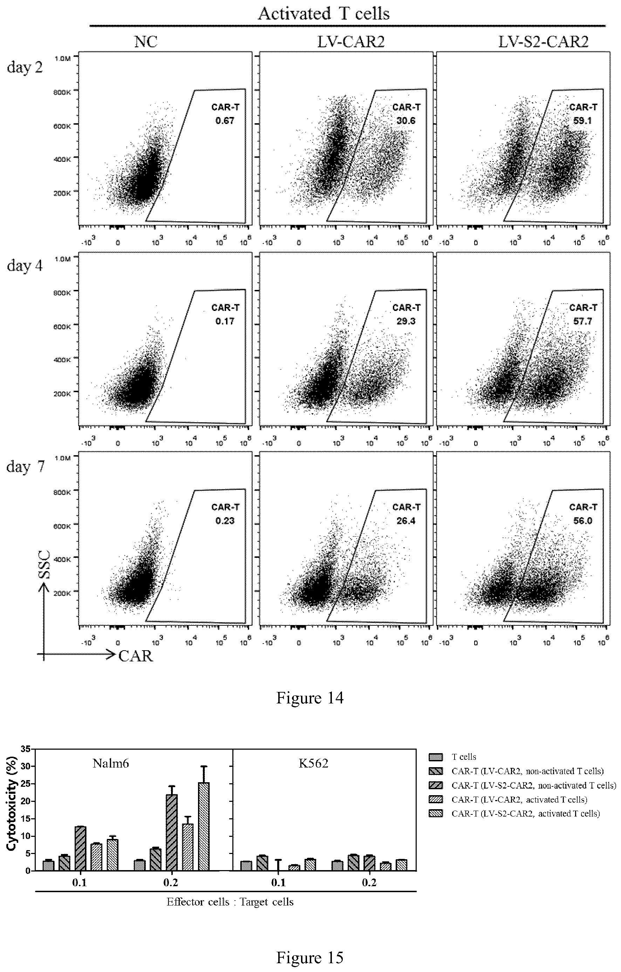

Floowing activation with CD3/CD28 magnetic beads for a duration of three days, both the LV-CAR2 and LV-S2-CAR2 vectors were introduced to the activated T cells at an MOI of 2. A Negative Control (NC) group was concurrently established, characterized by the addition of vehicle alone, devoid of any lentiviral vector, during the transduction phase. Subsequently, the expression positivity rates of the chimeric antigen receptor (CAR) in each experimental cohort were quantitatively assessed via flow cytometry at distinct temporal intervals post-transduction: namely, 2 days (Day 2), 4 days (Day 4), and 7 days (Day 7). Comparative analyses of the CAR expression positivity rates across all groups are graphically represented in Figure 14:

Depicted in Figure 14 is a comparative analysis delineating the transduction efficiencies of the LV-CAR2 and LV-S2-CAR2 vectors in activated T cells. Both vectors are capable of successfully transducing activated T cells, resulting in the generation of CAR-T cells characterized by stable CAR expression. However, the transduction efficacy of the LU-S2 lentiviral vector is notably superior-approximately double-that of the conventional lentiviral vector (LV).

4) Detection of in vitro CAR-T killing ability

The lactate dehydrogenase (LDH) assay was employed to assess the cytotoxic capabilities of CAR-T cells harvested on Day 4. The quantity of effector cells was determined according to the proportion of CAR-positive cells within each CAR-T cell population. Target cells comprised Nalm6 cells, notable for their CD19 positivity. Conversely, K562 cells, lacking CD 19 expression, served as the negative control for target cells. The CAR-T cell-negative control group was designated as the T cell group. The resultant data are illustrated in Figure 15.

As evidenced by the data in Figure 15, a comparison of in vitro CAR-T cell cytotoxicity reveals that, under equivalent effector-to-target ratios, CAR-T cells generated using the LV-S2-CAR2 vector exhibit superior killing capacity compared to those produced with the LV-CAR2 vector. This observation suggests that, beyond enhancing transduction positivity rates, the LU-S2 vector exerts additional, yet unidentified, effects that augment the tumor-killing potential of the CAR-T cells it facilitates. Particularly noteworthy is the heightened tumor-killing efficacy of CAR-T cells derived from unactivated T cells transduced with LV-S2-CAR2 under low effector-to-target ratios (0.1). Given that, in clinical settings, CAR-T cells and tumor cells often encounter each other at similarly low ratios, this finding implies that CAR-T cells generated from unactivated T cells using the LV-S2-CAR2 vector may exhibit superior clinical outcomes relative to conventional methods (i.e., CAR-T cells produced from activated T cells using LV vectors).

**Example 7: Comparative efficacy analysis of CAR-T cell products derived from diverse lentiviral vectors in mouse models**

a. Preparation of CAR-T samples via conventional protocol: Upon thawing cryopreserved T cells, activation was initiated using magnetic beads conjugated with CD3/CD28 antibodies. Post-activation, typically after a 72-hour period, the beads were carefully removed. Then the transduction enhancer Lentiboost was incorporated, followed by the introduction of LV-CAR2 lentiviral vectors to transduce the now-activated T cells. Approximately 2 to 7 days subsequent to transduction, CAR-T cells were subjected to cryopreservation. Throughout this timeline, the proportion of CAR-positive cells was quantified at various timepoints.

b. Novel process 1 (LV-S2-CAR2) for CAR-T sample preparation: Upon reviving cryopreserved T cells, they were cultivated for a 48-hour period under standard conditions. Thereafter, the transduction enhancer Lentiboost was introduced, followed by the application of LV-S2-CAR2 lentiviral vectors to transduce T cells in their unactivated state. Subsequent to a roughly 24-hour interval post-transduction, the CAR-T cells were expediently cryopreserved to ensure optimal preservation of cellular integrity and function.

c. Novel protocol 2 (LV-S2-CAR2) for CAR-T sample preparation: Following the revival of cryopreserved T cells, they underwent a 48-hour culture period to acclimate and recover. Subsequently, the transduction enhancer Lentiboost was introduced, followed by the application of LV-S2-CAR2 lentiviral vectors to transduce T cells in their unactivated state. Approximately 24 hours' post-transduction, the resultant CAR-T cells were cryopreserved.

d. After resuscitating the cryopreserved CAR-T cells prepared by novel process 1 and novel process 2, the CAR-T cells were cultured for 3-7 days, and the CAR positive rates were assessed respectively.

e. NCG female mice were intravenously injected with 1E+06 Nalm6-luciferase cells per mouse to establish a tumor model. After 5-7 days, tumor burden was detected by imaging in vivo, and the mice were divided into different groups based on the tumor burden, and the drugs were administered on the same day.

f. The samples prepared in a, b and c were revived and resuspended in PBS, to align the CAR-T positive cell ratios across samples, they were diluted with untransduced T cells; The experiment was structured into groups: G1 used PBS only; G2 comprised untransduced T cells; G3 reflected the conventional process (LV-CAR2) from sample a; G4 and G5 showcased two new processes (LV-S2-CAR2) from samples b and c, respectively. G5 also served as a blank control with no tumor cell inoculation. Ensuring consistency, groups G2 to G5 had equal T cell counts, and G3 to G5 had the same number of CAR-positive cells (1E+06 cells/mouse). This setup allowed for a fair assessment of the different CAR-T cell generation techniques.

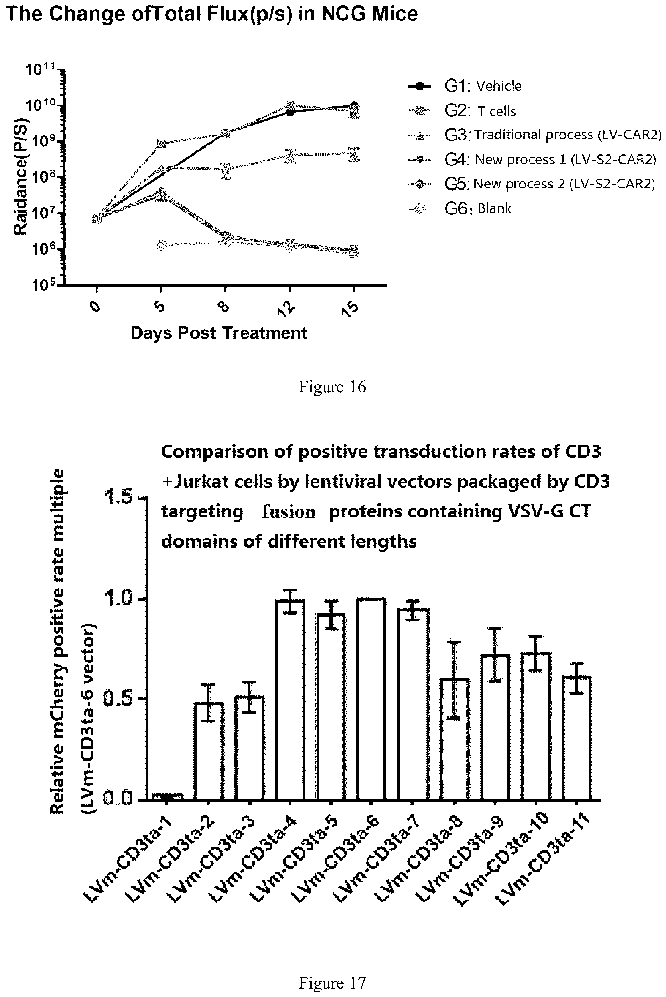

g. In vivo imaging was performed at day 5, 8, 12 and 15 after administration to detect the tumor burden of mice in each group, and a tumor burden curve was drawn. The results are shown in Figure 16.

According to the findings depicted in Figure 16, a comparison of the efficacy of CAR-T products prepared via differing lentiviral vector processes in mice reveals: under uniform experimental conditions, the effectiveness of CAR-T samples generated through two novel processes utilizing the targeted LV-S2-CAR2 lentiviral vector markedly surpasses that of samples prepared by conventional lentiviral methodologies known from prior art. This suggests that targeted lentiviral vectors, such as LU-S2-CAR2, confer unexpected benefits in the preparation of CAR-T products.

**Example 8: Comparative analysis of the functionality of lentiviral vectors encapsulating CD3-targeting fusion proteins, which incorporate VSV-G CT domains of varying lengths**

a. A series of lentiviruses encoding mCherry were packaged and used to transduce T cells through CD3-targeting

Plasmids were co-transfected into HEK-293T cells and packaged lentivirus according to Table 7.

Table 7: Lentivirus packaging

| Lentivirus | Types and ratios of lentiviral vectors |
|---|---|
| LVm-CD3ta-1 | pLV-mCherry: psPAX2: pMD2.G-Mut: pMD2.antiCD3-G-1 =2:1:1:0.5 |
| LVm-CD3ta-2 | pLV-mCherry: psPAX2: pMD2.G-Mut: pMD2.antiCD3-G-2 =2:1:1:0.5 |
| LVm-CD3ta-3 | pLV-mCherry: psPAX2: pMD2.G-Mut: pMD2.antiCD3-G-3 =2:1:1:0.5 |
| LVm-CD3ta-4 | pLV-mCherry: psPAX2: pMD2.G-Mut: pMD2.antiCD3-G-4 =2:1:1:0.5 |
| LVm-CD3ta-5 | pLV-mCherry: psPAX2: pMD2.G-Mut: pMD2.antiCD3-G-5 =2:1:1:0.5 |
| LVm-CD3ta-6 | pLU-mCherry: psPAX2: pMD2.G-Mut: pMD2.antiCD3-G-6=2:1:1:0.5 |
| LVm-CD3ta-7 | pLV-mCherry: psPAX2: pMD2.G-Mut: pMD2.antiCD3-G-7 =2:1:1:0.5 |
| LVm-CD3ta-8 | pLV-mCherry: psPAX2: pMD2.G-Mut: pMD2.antiCD3-G-8 =2:1:1:0.5 |
| LVm-CD3ta-9 | pLV-mCherry: psPAX2: pMD2.G-Mut: pMD2.antiCD3-G-9 =2:1:1:0.5 |
| LVm-CD3ta-10 | pLV-mCherry: psPAX2: pMD2.G-Mut: pMD2.antiCD3-G-10 =2:1:1:0.5 |
| LVm-CD3ta-11 | pLV-mCherry: psPAX2: pMD2.G-Mut: pMD2.antiCD3-G-11=2:1:1:0.5 |

In Table 7, pLV-mCherry signifies a transfer plasmid harboring the mCherry sequence; psPAX2 denotes a packaging plasmid that expresses lentiviral structural protein gag, non-structural protein pol, and serves as a plasmid for the regulatory protein rev; pMD2.G-Mut represents an envelope plasmid expressing a mutant envelope protein VSV-G (VSV-G-K47Q\R354Q); pMD2.antiCD3-G-1~11 is a plasmid coding for the fusion protein antiCD3-G-1-11, which encompasses an scFv targeting CD3 and the C-terminal domain of the VSV-G protein in varying lengths; herein, the anti-CD3 scFv exhibits an amino acid sequence delineated in SEQ ID NO:5; sequentially, the C-terminal domain of the VSV-G protein embedded within the fusion protein antiCD3-G-1~11 corresponds to the amino acid sequences spanning from positions 455 to 495, 445 to 495, 435 to 495, 425 to 495, 415 to 495, 405 to 495, 395 to 495, 385 to 495, 375 to 495, 365 to 495, and 355 to 495, respectively.

The C-terminal domain of the VSV-G protein contains the 455-495th (41 in total) amino acid of the VSV-G protein, and the amino acid sequence is shown in SEQ ID NO: 36.

IIGLIIGLFLVLRVGIHLCIKLKHTKKRQIYTDIEMNRLGK (SEQ ID NO: 36).

The C-terminal domain of the VSV-G protein contains the 445-495th (51 in total) amino acid of the VSV-G protein, and the amino acid sequence is shown in SEQ ID NO: 37.

WKSSIASFFFIIGLIIGLFLVLRVGIHLCIKLKHTKKRQIYTDIEMNRLGK (SEQ ID NO: 37).

The C-terminal domain of the VSV-G protein contains the 435-495th (61 in total) amino acid of the VSV-G protein, and the amino acid sequence is shown in SEQ ID NO: 38.

IELVEGWFSSWKSSIASFFFIIGLIIGLFLVLRVGIHLCIKLKHTKKRQIYTDIEM NRLGK (SEQ ID NO: 38).

The C-terminal domain of the VSV-G protein contains the 425-495th (71 in total) amino acid of the VSV-G protein, and the amino acid sequence is shown in SEQ ID NO: 39.

FGDTGLSKNPIELVEGWFSSWKSSIASFFFIIGLIIGLFLVLRVGIHLCIKLKHTK KRQIYTDIEMNRLGK (SEQ ID NO: 39).

The C-terminal domain of the VSV-G protein contains the 415-495th (81 in total) amino acid of the VSV-G protein, and the amino acid sequence is shown in SEQ ID NO: 40.

SQLPDDESLFFGDTGLSKNPIELVEGWFSSWKSSIASFFFIIGLIIGLFLVLRVGI HLCIKLKHTKKRQIYTDIEMNRLGK (SEQ ID NO: 40).

The C-terminal domain of the VSV-G protein contains 405-495th (91 in total) amino acid of the VSV-G protein, and the amino acid sequence is shown in SEQ ID NO: 13.

FEHPHIQDAASQLPDDESLFFGDTGLSKNPIELVEGWFSSWKSSIASFFFIIGLI IGLFLVLRVGIHLCIKLKHTKKRQIYTDIEMNRLGK (SEQ ID NO:13).

The C-terminal domain of the VSV-G protein contains the 395-495th (101 in total) amino acid of the VSV-G protein, and the amino acid sequence is shown in SEQ ID NO: 41.

DLHLSSKAQVFEHPHIQDAASQLPDDESLFFGDTGLSKNPIELVEGWFSSWK SSIASFFFIIGLIIGLFLVLRVGIHLCIKLKHTKKRQIYTDIEMNRLGK (SEQ ID NO: 41).

The C-terminal domain of the VSV-G protein contains the 385-495th (111 in total) amino acid of the VSV-G protein, and the amino acid sequence is shown in SEQ ID NO: 42.

YMIGHGMLDSDLHLSSKAQVFEHPHIQDAASQLPDDESLFFGDTGLSKNPIE LVEGWFSSWKSSIASFFFIIGLIIGLFLVLRVGIHLCIKLKHTKKRQIYTDIEMNRLGK (SEQ ID NO: 42).

The C-terminal domain of the VSV-G protein contains the 375-495th (121 in total) amino acid of the VSV-G protein, and the amino acid sequence is shown in SEQ ID NO: 43.

RTSSGYKFPLYMIGHGMLDSDLHLSSKAQVFEHPHIQDAASQLPDDESLFFG DTGLSKNPIELVEGWFSSWKSSIASFFFIIGLIIGLFLVLRVGIHLCIKLKHTKKRQIYTDIEM NRLGK (SEQ ID NO: 43).

The C-terminal domain of the VSV-G protein contains the 365-495th (131 in total) amino acid of the VSV-G protein, and the amino acid sequence is shown in SEQ ID NO: 44.

DVEIGPNGVLRTSSGYKFPLYMIGHGMLDSDLHLSSKAQVFEHPHIQDAASQ LPDDESLFFGDTGLSKNPIELVEGWFSSWKSSIASFFFIIGLIIGLFLVLRVGIHLCIKLKHTK KRQIYTDIEMNRLGK (SEQ ID NO: 44).

The C-terminal domain of the VSV-G protein contains the 355-495th (141 in total) amino acid of the VSV-G protein, and the amino acid sequence is shown in SEQ ID NO: 45.

ELWDDWAPYEDVEIGPNGVLRTSSGYKFPLYMIGHGMLDSDLHLSSKAQVF EHPHIQDAASQLPDDESLFFGDTGLSKNPIELVEGWFSSWKSSIASFFFIIGLIIGLFLVLRVG IHLCIKLKHTKKRQIYTDIEMNRLGK (SEQ ID NO: 45).

The amino acid sequence of fusion protein antiCD3-G-1 is shown in SEQ ID NO: 46.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTIIGLIIGLFLVLRVGIHLCIKLKHTKKRQIYTDIEMNRLGK (SEQ ID NO: 46).

The amino acid sequence of fusion protein antiCD3-G-2 is shown in SEQ ID NO: 47.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTWKSSIASFFFIIGLIIGLFLVLRVGIHLCIKLKHTKKRQIYT DIEMNRLGK (SEQ ID NO: 47).

The amino acid sequence of fusion protein antiCD3-G-3 is shown in SEQ ID NO: 48.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTIELVEGWFSSWKSSIASFFFIIGLIIGLFLVLRVGIHLCIKLK HTKKRQIYTDIEMNRLGK (SEQ ID NO: 48).

The amino acid sequence of fusion protein antiCD3-G-4 is shown in SEQ ID NO: 49.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTFGDTGLSKNPIELVEGWFSSWKSSIASFFFIIGLIIGLFLVLR VGIHLCIKLKHTKKRQIYTDIEMNRLGK (SEQ ID NO: 49).

The amino acid sequence of fusion protein antiCD3-G-5 is shown in SEQ ID NO: 50.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTSQLPDDESLFFGDTGLSKNPIELVEGWFSSWKSSIASFFFII GLIIGLFLVLRVGIHLCIKLKHTKKRQIYTDIEMNRLGK (SEQ ID NO: 50).

Wherein, antiCD3-G-6 is the fusion protein S2 and contains the amino acid sequence shown in SEQ ID NO: 15.
The amino acid sequence of fusion protein antiCD3-G-7 is shown in SEQ ID NO: 51.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTDLHLSSKAQVFEHPHIQDAASQLPDDESLFFGDTGLSKN PIELVEGWFSSWKSSIASFFFIIGLIIGLFLVLRVGIHLCIKLKHTKKRQIYTDIEMNRLGK (SEQ ID NO: 51).

The amino acid sequence of fusion protein antiCD3-G-8 is shown in SEQ ID NO: 52.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTYMIGHGMLDSDLHLSSKAQVFEHPHIQDAASQLPDDESL FFGDTGLSKNPIELVEGWFSSWKSSIASFFFIIGLIIGLFLVLRVGIHLCIKLKHTKKRQIYTD IEMNRLGK (SEQ ID NO: 52).

The amino acid sequence of fusion protein antiCD3-G-9 is shown in SEQ ID NO: 53.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTRTSSGYKFPLYMIGHGMLDSDLHLSSKAQVFEHPHIQDA ASQLPDDESLFFGDTGLSKNPIELVEGWFSSWKSSIASFFFIIGLIIGLFLVLRVGIHLCIKLK HTKKRQIYTDIEMNRLGK (SEQ ID NO: 53).

The amino acid sequence of fusion protein antiCD3-G-10 is shown in SEQ ID NO: 54.

DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYYDDHYCLDYW GQGTTLTVSSVEGGSGGSGGSGGSGGVDDIQLTQSPAIMSASPGEKVTMTCRASSSVSYM NWYQQKSGTSPKRWIYDTSKVASGVPYRFSGSGSGTSYSLTISSMEAEDAATYYCQQWS SNPLTFGAGTKLELKAAATTTDVEIGPNGVLRTSSGYKFPLYMIGHGMLDSDLHLSSKAQ VFEHPHIQDAASQLPDDESLFFGDTGLSKNPIELVEGWFSSWKSSIASFFFIIGLIIGLFLVLR VGIHLCIKLKHTKKRQIYTDIEMNRLGK (SEQ ID NO: 54).

The amino acid sequence of fusion protein antiCD3-G-11 is shown in SEQ ID NO: 55.

DIKLQQSGAELARPGASVKMSCKTSGYTFTRYTMHWVKQRPGQGLEWIGY

INPSRGYTNYNQKFKDKATLTTDKSSSTAYMQLSSLTSEDSAVYYCARYYDDHYCLDYW

GQGTTLTVSSVEGGSGGSGGSGGSGGVDDIQLTQSPAIMSASPGEKVTMTCRASSSVSYM

NWYQQKSGTSPKRWIYDTSKVASGVPYRFSGSGSGTSYSLTISSMEAEDAATYYCQQWS

SNPLTFGAGTKLELKAAATTTELWDDWAPYEDVEIGPNGVLRTSSGYKFPLYMIGHGML

DSDLHLSSKAQVFEHPHIQDAASQLPDDESLFFGDTGLSKNPIELVEGWFSSWKSSIASFF

FIIGLIIGLFLVLRVGIHLCIKLKHTKKRQIYTDIEMNRLGK (SEQ ID NO: 55).

b. Harvesting, purification and storage of lentiviral vectors

48-72 hours' post-transfection, the culture supernatant enriched with viral particles, was harvested. Subsequently, it was filtered through a 0.45 μm filter to remove cellular debris. The filtrate was then subjected to purification and concentration. Finally, the processed viral preparation was aliquoted, and stored in an ultralow-temperature refrigerator(<-75°C) for future use.

c. Lentivirus titer determination

The titer was measured after the frozen sample was taken out and thawed, the titer measurement and calculation refers to step 6.3 of Example 6.

d. All vectors were utilized to transduce CD3+ Jurkat cells at a multiplicity of infection (MOI) of 5. Following a period of 3 to 7 days, flow cytometry was employed to determine the percentage of cells expressing mCherry. The percentage of positive cells in each experimental group was normalized by dividing by that of the LUm-CD3ta-6 group. A histogram representing these relative percentages was constructed, which is illustrated in Figure 17.

Based on the findings presented in Figure 17, the transduction efficiency of CD3+ Jurkat cells using lentiviral vectors packaged with CD3-targeting fusion proteins that incorporate VSV-G C-terminal domains of varying lengths was assessed. Specifically, vectors LUm-CD3ta-4, LVm-CD3ta-5, LUm-CD3ta-6, and LUm-CD3ta-7 exhibited the highest transduction efficiency. In contrast, the transduction efficiency of other lentiviral vectors was notably lower. This observation indicates that the optimal length for the VSV-G C-terminal domain within the fusion protein is when it spans between the 386th and 434th amino acids, as well as up to the 495th amino acid of the envelope protein. Put differently, the ideal length for the VSV-G C-terminal domain should be longer than 61 amino acids but shorter than 111 amino acids. Under these conditions, the lentiviral vectors achieve the highest transduction efficiency.

**Example 9: Evaluation of titre variations amongst diverse CD3-targeted lentiviral vectors**

a. A series of lentiviruses encoding mCherry were packaged and used to transduce T cells through CD3-targeting

Plasmids were co-transfected into HEK-293T cells and packaged lentivirus according to Table 8.

Table 8: Lentiviral packaging

| Lentivirus | Types and ratios of lentiviral vectors |
| --- | --- |
| LV-S2 | pLV-mCherry: psPAX2: pMD2.G: pMD2.S2=2:1:1:0.5 |
| LV-2 | pLV-mCherry: psPAX2: pMD2.VSV-G-antiCD3 =2:1:1:0.5 |

pLV-mCherry signifies a transfer plasmid carrying mCherry sequence; psPAX2 denotes a packaging plasmid that expresses lentiviral structural protein gag, non-structural protein pol, and serves as a plasmid for the regulatory protein rev; pMD2.S2 is a plasmid that expresses a fusion protein, which comprises a single-chain variable fragment (scFv) targeting CD3 and the C-terminal domain of the VSV-G protein. Notably, the C-terminal domain of the VSV-G protein features an amino acid sequence shown in SEQ ID NO: 13, and the fusion protein itself contains the amino acid sequence shown in SEQ ID NO: 15; pMD2.VSV-G-antiCD3 is a plasmid expressing the fusion protein antiCD3 scFv-VSV-G, which incorporates a CD3-targeting scFv at the N-terminus of the full-length VSV-G protein, the fusion protein anti-CD3 scFv-VSV-G has an amino acid sequence shown in SEQ ID NO:56:

DIKLQQSGAELARPGASVKMSCKTSGYTFTRYTMHWVKQRPGQGLEWIGYINPSR

GYTNYNQKFKDKATLTTDKSSSTAYMQLSSLTSEDSAVYYCARYYDDHYCLDYWGQGT

TLTVSSVEGGSGGSGGSGGSGGVDDIQLTQSPAIMSASPGEKVTMTCRASSSVSYMNWY

QQKSGTSPKRWIYDTSKVASGVPYRFSGSGSGTSYSLTISSMEAEDAATYYCQQWSSNPL

TFGAGTKLELKAAATTKFTIVFPHNQKGNWKNVPSNYHYCPSSSDLNWHNDLIGTALQ

VKMPKSHKAIQADGWMCHASKWVTTCDFRWYGPKYITHSIRSFTPSVEQCKESIEQTKQ

GTWLNPGFPPQSCGYATVTDAEAVIVQVTPHHVLVDEYTGEWVDSQFINGKCSNYICPTV

HNSTTWHSDYKVKGLCDSNLISMDITFFSEDGELSSLGKEGTGFRSNYFAYETGGKACK

MQYCKHWGVRLPSGVWFEMADKDLFAAARFPECPEGSSISAPSQTSVDVSLIQDVERIL

DYSLCQETWSKIRAGLPISPVDLSYLAPKNPGTGPAFTIINGTLKYFETRYIRVDIAAPILSR

MVGMISGTTTERELWDDWAPYEDVEIGPNGVLRTSSGYKFPLYMIGHGMLDSDLHLSSK

AQVFEHPHIQDAASQLPDDESLFFGDTGLSKNPIELVEGWFSSWKSSIASFFFIIGLIIGLFL

VLRVGIHLCIKLKHTKKRQIYTDIEMNRLGK (SEQ ID NO: 56)

b. Lentivirus packaging and titer determination

The lentivirus packaging method refers to Example 1;
48-72 hours after transfection, following transfection, the culture supernatant, now laden with viral particles, was harvested. Subsequently, it was passed through a 0.45 $\mu$m filter to remove any cellular debris. The viral titer was then determined and calculated according to the procedures outlined in Step 6.3 of Example 6.

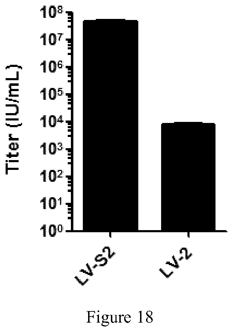

The titer results are illustrated in Figure 18. As the findings depicted in Figure 18, when compared to the lentiviral vector (LV-2), which is packaged utilizing a fusion protein where scFv is situated at the N-terminus of the complete VSV-G protein, there is a significant enhancement in the titer of the lentiviral vector (LV-S2). This improvement is observed in LV-S2, which is packaged by VSV-G in conjunction with a fusion protein containing scFv within the C-terminal domain of the VSV-G protein. Specifically, the titer of LV-2 stands at just 7.8E+03 TU/mL, whereas the titer of LV-S2 notably rises to 5.8E+07 TU/mL.

In addition, the usage of terms such as "first" and "second" serve solely descriptive functions, and should not be misconstrued as denoting or implying any precedence in significance or an implicit indication of the quantity of the technical features being referred to. Consequently, elements denoted by "first" or "second" may inherently encompass one or more instances of these features. Within the context of describing the present invention, the term "more" is utilized to signify a minimum of two, which could represent two, three, or any greater number, unless explicitly stated otherwise.

Throughout this specification, references to "an embodiment", "some embodiments", "one embodiment", "another example", "an example", "a specific example" or "some examples" indicate that the particular feature, structure, material, or characteristic described in connection with the embodiment or example is included in at least one embodiment or example of the present disclosure. Thus, the occurrences of the aforementioned phrases throughout this specification are not necessarily refer to the same embodiment or example of the present disclosure. Furthermore, the specific features, structures, materials, or characteristics may be combined in any suitable manner in one or more embodiments or examples, as would be apparent to those skilled in the art. while various embodiments and examples may be presented separately, it is within the capability of those skilled in the art to combine elements from different embodiments or examples provided there is no conflict or inconsistency in doing so.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

**Claims**

1. A group of viral vectors, comprising:

   a first viral vector, wherein the first viral vector carries a first nucleic acid molecule, and the first nucleic acid molecule encodes an envelope protein;
   at least a second viral vector, wherein the second viral vector carries a second nucleic acid molecule, the second nucleic acid molecule encodes at least one fusion protein, the fusion protein includes at least one single chain antibody and the C-terminal domain of the envelope protein; the single chain antibody is capable of binding to CD28 or CD3, the C-terminal domain of the envelope protein includes a transmembrane region and a intracellular region of the envelope protein, the C-terminal of the at least one single chain antibody is connected to the N-terminal of the C-terminal domain of the envelope protein;
   the first nucleic acid molecule and the second nucleic acid molecule are arranged to express the envelope protein and the fusion protein, and the envelope protein and the fusion protein are in a non-fusion form.

2. The viral vectors according to claim 1, wherein the viral vectors are retroviral vectors, lentiviral vectors or other enveloped viral vectors.

3. The viral vectors according to claim 2, wherein the enveloped virus comprises at least one selected from the group consisting of Bornaviridae, Nyamaviridae, Arenaviridae, Filoviridae, Hantaviridae, Nairoviridae, Orthomyxoviridae, Paramyxoviridae, Bunyaviridae, Phenuiviridae, Rhabdoviridae, Arteriviridae, Coronaviridae, Flaviviridae, Togaviridae, Hepadnaviridae, Spumavirus, Iridoviridae, Herpesviridae, Poxviridae and Deltavirus;

   optionally, the envelope protein is an envelope G glycoprotein or a mutant variant thereof from a vesicular stomatitis virus belonging to the family Rhabdoviridae;
   optionally, the envelope G glycoprotein has an amino acid sequence shown in SEQ ID NO:1.

4. The viral vectors according to claim 3, wherein the mutant of the envelope protein has a mutation that weakens the attachment capacity;

   optionally, the mutant of the envelope G glycoprotein has K47Q and R354Q mutations;
   optionally, the mutant of the envelope G glycoprotein has an amino acid sequence shown in SEQ ID NO: 2.

5. The viral vectors according to claim 1, wherein the single chain antibody is capable of binding to CD28, optionally, the single chain antibody has an amino acid sequence shown in SEQ ID NO: 3 or 4;

   optionally, the single chain antibody is capable of binding to CD3, optionally, the single chain antibody has an amino acid sequence shown in SEQ ID NO: 5 or 6;
   optionally, the fusion protein comprises a first single chain antibody, a second single chain antibody and a C-terminal domain of the envelope protein. The first single chain antibody is capable of binding to CD28, and the second single chain antibody is capable of binding to CD3, the C-terminal of the first single chain antibody is connected to the N-terminal of the second single chain antibody, and the C-terminal of the second single chain antibody is connected to the N-terminal of the C-terminal domain of the envelope protein; or, the C-terminal of the second single chain antibody is connected to the N-terminal of the first single chain antibody, and the C-terminal of the first single chain antibody is connected to the N-terminal of the C-terminal domain of the envelope protein.
   optionally, the first single chain antibody has an amino acid sequence shown in SEQ ID NO: 3 or 4;
   optionally, the second single chain antibody has an amino acid sequence shown in SEQ ID NO: 5 or 6;
   optionally, the C-terminal domain of the envelope protein further comprises at least a portion of the extracellular region of the envelope protein.

6. The viral vectors according to any one of claims 1~5, wherein the fusion protein further comprises a first linking peptide, wherein the N-terminal of the first linking peptideis connected to the C-terminal of the first single chain antibody, and the C-terminal of the first linking peptide is connected to the N-terminal of the second single chain antibody; or, the N-terminal of the first linking peptide is connected to the C-terminal of the second single chain antibody, and the C-terminal of the first linking peptide is connected to the N-terminal of the first single chain antibody;

optionally, the first linking peptide has an amino acid sequence shown in SEQ ID NO: 7, 8, 9, 10 or 11;

optionally, the fusion protein further comprises a second linking peptide, the N-terminal of the second linking peptide is connected to the C-terminal of the at least one single chain antibody, and the C-terminal of the second linking peptide is connected to the N-terminal of the C-terminal domain of the envelope protein;

optionally, the second linking peptide has an amino acid sequence shown in SEQ ID NO: 12.

7. The viral vectors according to any one of claims 1~6, wherein the C-terminal domain of the envelope protein comprises a peptide chain, the peptide chain starts from an amino acid between the 386th and the 434th amino acid, to the 495th amino acid of the envelope protein;

preferably, the C-terminal domain of the envelope protein comprises a peptide chain, the peptide chain starts from an amino acid between the 395th and the 425th amino acid, to the 495th amino acid of the envelope protein;

optionally, the C-terminal domain of the envelope protein comprises the 425-495th amino acid, the 415-495th amino acid, the 405-495th amino acid, or the 395-495th amino acid of the VSV-G protein;

optionally, the C-terminal domain of the envelope protein has an amino acid sequence shown in SEQ ID NO: 13, 39, 40 or 41;

optionally, the fusion protein has an amino acid sequence shown in SEQ ID NO:14, 15, 16, 17, 18, 19 or 20.

8. The viral vectors according to claim 1, wherein the viral vector further comprises:

a first promoter, which is operably linked to the first nucleic acid molecule; and
a second promoter, which is operably linked to the second nucleic acid molecule.

9. The viral vectors according to claim 8, wherein each of the first promoter and the second promoter is independently selected from CMV, EF-1 or RSV promoters.

10. The viral vectors according to claim 1, wherein the first nucleic acid molecule has a nucleotide sequence shown in SEQ ID NO: 21 or 35;

optionally, the second nucleic acid molecule has a nucleotide sequence shown in SEQ ID NO: 22, 23, 24, 25, 32, 33 or 34;

optionally, the second nucleic acid molecule further comprises a nucleic acid sequence encoding a signal peptide;

optionally, the nucleic acid sequence encoding a signal peptide has a nucleotide sequence shown in SEQ ID NO: 26;

optionally, the ratio of the copy number of the first nucleic acid molecule and the second nucleic acid molecule is 1:1 ~ 4:1.

11. The viral vectors according to claim 1, wherein the first viral vector and the second viral vector are the same vector.

12. The viral vectors according to claim 11, wherein the viral vector further comprises:
an internal ribosome entry site sequence, wherein the internal ribosome entry site sequence is arranged between the first nucleic acid molecule and the second nucleic acid molecule.

13. The viral vectors according to claim 11, wherein the viral vector further comprises:
a third nucleic acid molecule, which is arranged between the first nucleic acid molecule and the second nucleic acid molecule, and the third nucleic acid molecule encodes a third linking peptide, and the third linking peptide can be cleaved.

14. The viral vectors according to claim 1, wherein the first viral vector and the second viral vector are pMD2.G, pCMV, pMD2.G mutant or pCMV mutant.

15. The viral vectors according to claim 1, wherein the viral vectors further comprises: a third viral vector and a fourth viral vector, the third viral vector carries gene of interest, and the fourth viral vector carries the viral structural protein genes, and viral packaging enzyme gene and optional regulatory factor rev gene;

optionally, the structural protein genes, the viral packaging enzyme gene and the regulatory factor rev gene are arranged in the same fourth viral vector or different fourth viral vectors;

optionally, the viral packaging enzyme comprises at least one of reverse transcriptase, protease, and integrase.

16. The viral vectors according to claim 15, wherein the third viral vector is a transfer vector, the transfer vector comprises a lentivirus packaging signal,

   optionally, the lentivirus packaging signal comprises: Ψ;
   optionally, the transfer vector is pLV;
   optionally, the fourth viral vector is psPAX2.

17. The viral vectors according to claim 15 or 16, wherein the gene of interest is a nucleic acid molecule encoding a chimeric antigen receptor.

18. A method for obtaining lentivirus, comprising: introducing the viral vectors according to any one of claims 1~17 into a first recipient cell; culturing the first recipient cell to obtain a virus.

19. The method according to claim 18, wherein the virus is lentivirus, the first viral vector and the second viral vector are different vectors, the mass ratio of the third viral vector, the fourth viral vector, the first viral vector and the second viral vector is 1: 1: 1: 0.25 ~ 2: 1: 1: 1,

   preferably, the mass ratio of the third viral vector, the fourth viral vector, the first viral vector and the second viral vector is 2: 1: 1: 0.5;
   preferably, the mass ratio of the third viral vector, the fourth viral vector, the first viral vector and the second viral vector is 1: 1: 1: 0.5;
   preferably, the mass ratio of the third viral vector, the fourth viral vector, the first viral vector and the second viral vector is 1: 1: 1: 1;
   optionally, the first recipient cell is 293 T.

20. A lentivirus, wherein the lentivirus is obtained by packaging according to the method of claim 18 or 19.

21. A lentivirus, wherein the lentivirus expresses an envelope protein and a fusion protein, wherein the fusion protein comprises at least one single chain antibody and a C-terminal domain of the envelope protein, the single chain antibody is capable of binding to CD28 or CD3, the C-terminal domain of the envelope protein comprises transmembrane and intracellular regions of the envelope protein, the C-terminal of the at least one single chain antibody is connected to the N-terminal of the C-terminal domain of the envelope protein,
optionally, the envelope protein is an envelope G glycoprotein or a mutant of envelope G glycoprotein of vesicular stomatitis virus.

22. A lentivirus, wherein the lentivirus expresses an envelope protein and a fusion protein, wherein the fusion protein comprises a first single chain antibody, a second single chain antibody and a C-terminal domain of the envelope protein, the first single chain antibody is capable of binding to CD28, the second single chain antibody is capable of binding to CD3, the C-terminal domain of the envelope protein comprises a transmembrane region and a intracellular region of the envelope protein, the C-terminal of the first single chain antibody is connected to the N-terminal of the second single chain antibody, and the C-terminal of the second single chain antibody is connected to the N-terminal of the C-terminal domain of the envelope protein; or, the C-terminal of the second single chain antibody is connected to the N-terminal of the first single chain antibody, and the C-terminal of the first single chain antibody is connected to the N-terminal of the C-terminal domain of the envelope protein,
optionally, the envelope protein is an envelope G glycoprotein or a mutant of envelope G glycoprotein of vesicular stomatitis virus.

23. A method for introducing lentivirus into unactivated T lymphocytes, wherein the lentiviral vectors according to any one of claims 1~17 are used to electroporate or transfect the unactivated T lymphocytes, or the lentivirus according to any one of claims 20~22 are used to transduce the unactivated T lymphocytes.

24. A method for expressing gene of interest, comprising:

   introducing the viral vectors according to any one of claims 1~17 or lentivirus according to any one of claims 20~22 integrated with the gene of interest into the second recipient cell;
   culturing the second recipient cell into which the viral vector or lentivirus is introduced to express the gene of interest;
   optionally, the introduction into the second recipient cell is carried out by electrotransfection, transfection or infection;

optionally, the second receptor cell is a T cell.

25. A method of obtaining CAR-T cells, comprising:

introducing the viral vectors according to any one of claims 1~17 or lentivirus according to any one of claims 20~22 integrated with chimeric antigen receptor encoding nucleic acid into T lymphocytes;

culturing the T lymphocytes into which the viral vectors or lentivirus is introduced to express chimeric antigen receptor;

optionally, the introduction into the T lymphocytes is carried out by electrotransfection, transfection or infection.

26. A CAR-T cell, wherein the CAR-T cell is prepared according to the method of claim 25.

27. A pharmaceutical composition comprising the viral vectors according to any one of claims 1~17, the lentivirus according to any one of claims 20~22, or the CAR-T cell according to claim 26.

28. Use of the viral vectors according to any one of claims 1~17, the lentiviruses according to any one of claims 20~22, the CAR-T cells according to claim 26 or the pharmaceutical compositions according to claim 27 to activate immunity or treat or prevent diseases.

29. Use of the viral vectors according to any one of claims 1~17, the lentiviruses according to any one of claims 20~22, the CAR-T cells according to claim 26 or the pharmaceutical compositions according to claim 27 to treat or prevent tumors.

30. A method of activating immunity or treating or preventing diseases comprising administering to the subject a therapeutically effective amount of the viral vectors according to any one of claims 1~17, the lentiviruses according to any one of claims 20~22, the CAR-T cells according to claim 26 or the pharmaceutical compositions according to claim 27.

31. The method according to claim 31, wherein the diseases are tumors.

anti-CD28/3-G: NH₂- [anti-CD28 scFv] [anti-CD3 scFv] [VSV-G C-terminal] -COOH

VSV-G      The first      The second
signal peptide    linking peptide    linking peptide

anti-CD3-G: NH₂- [anti-CD3 scFv] [VSV-G C-terminal] -COOH

VSV-G      The second
signal peptide    linking peptide

Figure 1

pMD2.G-Mut
5822 bp

Figure 2

Figure 3

Figure 4

Figure 5

Figure 6

Figure 7

Figure 8

Figure 9

Figure 10

Figure 11

Figure 12

Figure 13

Figure 14

Figure 15

## The Change ofTotal Flux(p/s) in NCG Mice

Figure 16

Figure 17

Figure 18

## INTERNATIONAL SEARCH REPORT

| International application No. |
| --- |
| **PCT/CN2023/072446** |

**A. CLASSIFICATION OF SUBJECT MATTER**

C12N 15/867(2006.01)i;C12N15/47(2006.01)i;C12N15/62(2006.01)i; C12N7/01(2006.01)i;
A61K39/205(2006.01)i;A61K47/68(2017.01)i;A61K48/00(2006.01)i;A61P31/14(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNTXT, DWPI, USTXT, EPTXT, WOTXT , ENTXT, CNKI, 万方数据库, WANFANG DATABASE, WEB OF SCIENCE, PUBMED: 病毒载体, 慢病毒, 反转录病毒, 包膜蛋白, 融合蛋白, 单链抗体, 跨膜区, C-端结构域, 胞内区, 假型化, 突变体, viral vector, Lentiviral, Retrovirus, Envelop protein, VSV-G, pMD2.G-Mut, pMD2.G, K47Q, R354Q, fusion protein, scFv, single-chain antibody, C-terminal domain, transmembrane region, intracellular region, pseudotyped, mutant; GenBank+EMBL +中国专利生物序列检索系统: 对SEQ ID NOs: 1-26, 32-35的序列检索, GenBank+EMBL+China Patent Biological Sequence Search System: search for SEQ ID NOs: 1-26, 32-35

**C. DOCUMENTS CONSIDERED TO BE RELEVANT**

| Category* | Citation of document, with indication, where appropriate, of the relevant passages | Relevant to claim No. |
| --- | --- | --- |
| PX | CN 114317609 A (GUANGDONG DONGSUNANG PHARMACEUTICAL CO., LTD.) 12 April 2022 (2022-04-12) claims 1-22, description paragraphs 52-57 | 1-31 |
| A | US 2016333374 A1 (SIRION BIOTECH GMBH et al.) 17 November 2016 (2016-11-17) see claims 1-21, description paragraph 28, embodiments 2, 4 | 1-31 |
| A | CN 111315761 A (MILTENYI BIOTEC GMBH) 19 June 2020 (2020-06-19) see claims 1-15, description paragraph 6 | 1-31 |
| A | CN 104080917 A (INSTITUT NATIONAL DE LA SANTE ET DE LA RECHERCHE MEDICALE (INSERM) et al.) 01 October 2014 (2014-10-01) see claims 1-15 | 1-31 |

☑ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **02 May 2023** | **05 May 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/072446**

**C.    DOCUMENTS CONSIDERED TO BE RELEVANT**

| Category* | Citation of document, with indication, where appropriate, of the relevant passages | Relevant to claim No. |
|---|---|---|
| A | 赵朴 等 (ZHAO, Pu et al.). "假型病毒载体研究进展 (Progress in Research of Pseudotyped Viral Vectors)" <br> 生物技术通报 (Biotechnology Bulletin), <br> Vol. 2008, No. (1), 26 February 2008 (2008-02-26), 69-71 <br> ISSN: 1002-5464, <br>       pp. 67-71, see text, section 1 | 1-31 |
| A | DREJA, H. et al. "The Effects of N-terminal Insertion into VSV-G of an scFv Peptide" <br> Virol J, Vol. 2006, No. (3), 02 September 2006 (2006-09-02), document number 69 <br> ISSN: 1743-422X, <br>       document number 69, see abstract | 1-31 |

Form PCT/ISA/210 (second sheet) (July 2022)

## INTERNATIONAL SEARCH REPORT

| International application No. |
|---|
| **PCT/CN2023/072446** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑   forming part of the international application as filed.

     b.    ☐   furnished subsequent to the international filing date for the purposes of international search (Rule 13*ter*.1(a)),

           ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2023/072446**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **30-31**
   because they relate to subject matter not required to be searched by this Authority, namely:

   Claims 30-31 include a method for treatment of the living human or animal body, which belongs to the cases set forth in PCT Rule 39(iv) for which a search is not required. In the present report, a search is performed for the amended subject matter that might be reasonably expected.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.

**PCT/CN2023/072446**

| Patent document cited in search report | | | Publication date (day/month/year) | Patent family member(s) | | | Publication date (day/month/year) |
|---|---|---|---|---|---|---|---|
| CN | 114317609 | A | 12 April 2022 | WO | 2022068837 | A1 | 07 April 2022 |
| US | 2016333374 | A1 | 17 November 2016 | US | 10072273 | B2 | 11 September 2018 |
| | | | | CA | 2935929 | A1 | 16 July 2015 |
| | | | | CA | 2935929 | C | 22 February 2022 |
| | | | | ES | 2761638 | T3 | 20 May 2020 |
| | | | | EP | 3670651 | A1 | 24 June 2020 |
| | | | | WO | 2015104376 | A1 | 16 July 2015 |
| | | | | EP | 3092306 | A1 | 16 November 2016 |
| | | | | EP | 3092306 | B1 | 16 October 2019 |
| | | | | JP | 2017505606 | A | 23 February 2017 |
| | | | | JP | 6412579 | B2 | 24 October 2018 |
| CN | 111315761 | A | 19 June 2020 | CA | 3080424 | A1 | 09 May 2019 |
| | | | | JP | 2021500909 | A | 14 January 2021 |
| | | | | JP | 7237960 | B2 | 13 March 2023 |
| | | | | EP | 3704136 | A1 | 09 September 2020 |
| | | | | KR | 20200074204 | A | 24 June 2020 |
| | | | | WO | 2019086351 | A1 | 09 May 2019 |
| | | | | US | 2021180083 | A1 | 17 June 2021 |
| CN | 104080917 | A | 01 October 2014 | RU | 2014117201 | A | 10 November 2015 |
| | | | | RU | 2618864 | C2 | 11 May 2017 |
| | | | | ES | 2642276 | T3 | 16 November 2017 |
| | | | | EP | 2761010 | A1 | 06 August 2014 |
| | | | | EP | 2761010 | B1 | 05 July 2017 |
| | | | | WO | 2013045639 | A1 | 04 April 2013 |
| | | | | CA | 2849045 | A1 | 04 April 2013 |
| | | | | CA | 2849045 | C | 17 November 2020 |
| | | | | US | 2014235700 | A1 | 21 August 2014 |
| | | | | US | 9249426 | B2 | 02 February 2016 |

Form PCT/ISA/210 (patent family annex) (July 2022)